(19) Europäisches Patentamt / European Patent Office / Office européen des brevets

(11) **EP 4 646 996 A1**

(12) **EUROPÄISCHE PATENTANMELDUNG**

(43) Veröffentlichungstag:
**12.11.2025 Patentblatt 2025/46**

(21) Anmeldenummer: **24174607.2**

(22) Anmeldetag: **07.05.2024**

(51) Internationale Patentklassifikation (IPC):
**A61B 3/00** (2006.01)   **A61B 5/00** (2006.01)

(52) Gemeinsame Patentklassifikation (CPC):
**A61B 3/0008**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Oertli-Instrumente AG**
**9442 Berneck (CH)**

(72) Erfinder:
• **HITZ, Daniel**
**9063 Stein AR (CH)**

• **SCHMIDBERGER, Bernhard**
**88299 Leutkirch im Allgäu (DE)**
• **SANSEVERINO, Flavio**
**9445 Rebstein (CH)**
• **BRILL, Norbert**
**8280 Kreuzlingen (CH)**

(74) Vertreter: **Piticco, Lorena**
**Isler & Pedrazzini AG**
**Giesshübelstrasse 45**
**Postfach 1772**
**8027 Zürich (CH)**

(54) **OPHTHALMISCHE BELEUCHTUNGSVORRICHTUNG ZUR BELEUCHTUNG EINES OPHTHALMISCHEN LICHTINSTRUMENTS**

(57) Eine ophthalmische Beleuchtungsvorrichtung (1) zur Beleuchtung eines ophthalmischen Lichtinstruments (2) umfasst mindestens ein Leuchtelement (3) und mindestens eine Anregungsquelle (4). Die Anregungsquelle (4) ist zur Anregung des Leuchtelements (3) mittels Anregungslicht (5) aus stimulierter Emission ausgebildet. Das Leuchtelement (3) ist dazu ausgebildet, nach Anregung zu photolumineszieren. Die ophthalmische Beleuchtungsvorrichtung (1) ist dazu ausgebildet, das ophthalmische Lichtinstrument (2) mittels Beleuchtungslicht (6) umfassend oder bestehend aus der Photolumineszenz (7) des Leuchtelements (3) und/oder stimulierter Emission (5) zu beleuchten.

FIG. 1

EP 4 646 996 A1

**Beschreibung**

TECHNISCHES GEBIET

Die vorliegende Erfindung betrifft eine ophthalmische Beleuchtungsvorrichtung gemäss den Ansprüchen 1, 13 und 14 sowie ein ophthalmisches Beleuchtungssystem umfassend eine derartige ophthalmische Beleuchtungsvorrichtung und ein ophthalmisches Lichtinstrument.

STAND DER TECHNIK

Die intraokulare Beleuchtung bei Vitrektomien und anderen chirurgischen Eingriffen im Auge ist von zentraler Bedeutung. Zur Beleuchtung des Operationsfeldes werden typischerweise ophthalmologische Beleuchtungssysteme umfassend eine ophthalmische Beleuchtungsvorrichtung und ein ophthalmisches Lichtinstrument zum Zuleiten von Licht an die chirurgische Stelle im Auge verwendet. Ein Benutzer, beispielsweise ein Chirurg oder ein anderes medizinisches Fachpersonal, kann eine Beleuchtungssonde in das Auge einführen, um das Innere des Auges für einen Eingriff zu beleuchten. Typischerweise wird die Sonde an einen optischen Anschluss eines ophthalmologischen Beleuchtungssystems angeschlossen.

Der Chirurg wünscht sich von der Beleuchtung eine ausreichende Helligkeit für alle aktuellen Lichtinstrumente (z.B. 27G/25G/23G/20G), um Gewebe und Strukturen im Auge sichtbar zu machen. Zusätzlich soll die Ausleuchtung im Auge breit und möglichst homogen sein, um auch in peripheren Netzhautregion allfällige Ereignisse beobachten zu können. Schatten- oder Farbringe wirken für den Chirurgen störend. Grundsätzlich wird die Farbe Weiss für die Visualisierung des Gewebes und der Strukturen verwendet. Zusätzlich können Farbtöne wie z.B. Weiss mit Blaustich oder Weiss mit Gelbstich respektive Weiss mit Grünstich in gewissen Operationsschritten eine noch bessere Sichtbarkeit erzielen. Darüber hinaus soll die Leuchtquelle ausreichend Licht für Lichtinstrumente mit Fasern ≤200 μm bereitstellen, um die klinische Anwendung von 29G Lichtinstrumenten sowie beleuchteter Spezialinstrumente zu ermöglichen.

Dabei stellen sich verschiedenste Anforderungen an die Beleuchtungsvorrichtungen solcher Beleuchtungssysteme. Beispielsweise soll die Beleuchtungsvorrichtung ausreichend Lichtstrom (insbesondere ≥20 lm) liefern, so dass auch 27G Lichtinstrumente ausreichend beleuchtet werden. Dies ist insbesondere in kritischen Situationen wie beispielsweise einer Netzhautblutung von grosser Bedeutung. Zudem sollte die Beleuchtungsvorrichtung ausreichend Lichtstrom für Lichtinstrumente mit Fasern ≤200 μm liefern, sodass 29G-Vitrektomien ermöglicht werden, sowie beleuchtete Instrumente sollten ausreichend Licht zur Verfügung stellen. Beispielsweise wäre ein Lichtstrom von ≥10 lm in 200 μm Fasern wünschenswert. Der Lichtstrom von bekannten Beleuchtungsvorrichtungen wie z.B. LED, XENON-Lampen, Halogen etc., ist für minimalinvasive Eingriffe mit Lichtinstrumenten kleiner 27G allerdings zu gering. Demgegenüber steht der zukünftige Trend zu noch kleineren Inzisionen in der Vitrektomie wie 29G. Diese sind jedoch mit den heutigen Beleuchtungstechnologien noch nicht umsetzbar, da keine ausreichende Helligkeit im Patientenauge erlangt werden kann. Weiter kann der Abbildungsmassstab unzureichend sein oder die numerische Apertur (NA) der Faser wird überschritten. Herkömmliche Beleuchtungsvorrichtungen verwenden oftmals Kugelstrahler, welche durch Optiken auf das proximale Ende der Faser fokussiert werden. Dabei muss der Kugelstrahler durch das optische System auf die NA der Faser (bildseitiger Abstrahlwinkel) abgestimmt werden. In der paraxialen Optik definiert das Verhältnis aus objektseitigem Abstrahlwinkel (Beleuchtungsvorrichtung, bspw. LED-Chip) und bildseitigem Abstrahlwinkel (NA der Faser) den Abbildungsmassstab (=M). Es gilt:

$$M = \frac{\varphi}{\varphi'}$$

mit $\varphi$ bzw. $\varphi'$: objekt- bzw. bildseitiger Abstrahlwinkel.

Kugelstrahler weisen hohe Abstrahlwinkel auf wohingegen Lichtfasern eher kleine Abstrahlwinkel benötigen (Grenzwinkel der Totalreflexion). Folglich ergibt sich ein hoher Abbildungsmassstab. Bei grossem Abbildungsmassstab wird die Faser jedoch überstrahlt, wodurch Lichtstrom verloren geht. Folglich sollten die Abstrahlwinkel und der Abbildungsmassstab aufeinander abgestimmt sein.

Weiter ist die thermische Stabilität von Kunststofflichtfasern begrenzt. Vor allem bei grossen Lichtfasern (23G) kann der einkoppelbare Lichtstrom so gross werden, dass die Fasern thermisch beschädigt werden können. Bei kleineren Faserdurchmessern hingegen kann zu wenig Lichtstrom eingekoppelt werden, da zu viel Lichtstrom der Beleuchtungsvorrichtung im optischen Pfad verloren geht. Die entgegengesetzten Anforderungen von ausreichend Lichtstrom in kleinen Faserdurchmessern versus zu hohen Lichtstrom bei grossen Faserdurchmessern kann erfüllt werden, wenn eine Erkennung des Lichtinstruments (Gauge-Grösse) implementiert ist. Eine Erkennung des Lichtinstrumentes ist aus Sicht der Patientensicherheit notwendig.

Um unterschiedliche Strukturen im Auge wie Glaskörper, Netzhaut, etc. hervorzuheben, ist es bekannt, die Farbtöne der Beleuchtungsvorrichtungen von Blau bis Gelb stufenlos einzustellen. Dadurch können jedoch Farbinhomogenitäten wie Farbringe und -unterschiede in Lichtkegel auftreten, was auf eine chromatische Aberration bei der Verwendung von Beleuchtungsvorrichtungen mit unterschiedlichen Wellenlängen zurückzuführen ist.

Weiter soll eine phototoxische Dosis im Patientenauge weitmöglichst verringert werden. Zur Berechnung der phototoxischen Dosis im Patientenauge ist allerdings die Erkennung der Gauge-Grösse des Lichtinstruments und die Aufzeichnung von Lichtstrom und Bestrahlungsdauer notwendig. Eine Identifikation der Gauge-Grösse des Lichtinstruments fehlt jedoch in den gängigen Beleuchtungsvorrichtungen. Als Folge davon wird bei unterschiedlichen Lichtinstrumenten (unterschiedliche Faserdurchmesser) ein unterschiedlich hoher Lichtstrom eingekoppelt. Zudem macht ein hoher Lichtstrom bei Lichtinstrumenten mit hohem Faserdurchmesser eine Lichtstrombegrenzung notwendig, da ansonsten eine thermische Schädigung durch hohen Lichtstrom in grossen Lichtfasern resultieren kann. Auch ist bei den gängigen Beleuchtungsvorrichtungen keine Überwachung des Lichtstroms vorhanden. Stattdessen wird der Lichtstrom der Beleuchtungsvorrichtung nur indirekt durch deren Bestromung erfasst.

All dies wirkt für den Chirurgen respektive die Chirurgin sehr störend und kann sich nachteilig auf den ophthalmischen Eingriff und somit auf die Patientensicherheit auswirken.

In der US 2013/0265548 A1 wird ein ophthalmologisches Endoilluminationssystem offenbart, welches ein Handstück mit bis zu drei Laserdioden offenbart, welche in eine optische Faser gekoppelt werden. Die optische Faser ist in eine Kanüle eingesetzt. Für eine Beleuchtung können die drei Dioden über eine Linse auf die Faser fokussiert werden. Daneben werden auch Varianten mit je einer separaten Faser je Laserdiode offenbart. Bei Kombination von einer grünen, einer roten und einer blauen Laserdiode kann im Auge weisses Licht erzeugt werden. Weiter wird ein Maximum von 11 Lumen ohne Angabe der Gauge Grösse des Lichtinstruments beschrieben. Damit erreicht die beschriebene Lichtstrommenge nicht mehr für grössere Gauge Grössen (> 27G) aus. Weitere Probleme sind wahrscheinlich Speckling-Effekte, welche durch die Verwendung von drei monochromatischen Laserdioden entstehen, sowie hohe Herstellkosten.

## DARSTELLUNG DER ERFINDUNG

Es ist eine Aufgabe der vorliegenden Erfindung, eine ophthalmische Beleuchtungsvorrichtung zur Beleuchtung eines ophthalmischen Lichtinstruments anzugeben, welches eine verbesserte und insbesondere auch medizinisch sichere Beleuchtung einer chirurgischen Stelle im Auge eines Patienten ermöglicht.

Diese Aufgabe wird durch eine ophthalmische Beleuchtungsvorrichtung gemäss den Ansprüchen 1, 13 und 14 gelöst.

In einem ersten Aspekt wird also eine ophthalmische Beleuchtungsvorrichtung zur Beleuchtung eines ophthalmischen Lichtinstruments angegeben, welche mindestens ein Leuchtelement und mindestens eine Anregungsquelle umfasst. Die Anregungsquelle ist zur Anregung des Leuchtelements mittels Anregungslicht aus stimulierter Emission ausgebildet. Das Leuchtelement ist dazu ausgebildet, nach Anregung zu photolumineszieren. Die ophthalmische Beleuchtungsvorrichtung ist dazu ausgebildet, das ophthalmische Lichtinstrument mittels Beleuchtungslicht umfassend oder bestehend aus der Photolumineszenz des Leuchtelements und/oder stimulierter Emission zu beleuchten.

Das Leuchtelement ist also photolumineszierend. Unter Photolumineszenz respektive photolumineszierend wird die spontane Emission von elektromagnetischer Strahlung des durch Photonen angeregten Leuchtelements verstanden, wenn dieses von seinem angeregten Zustand in einen weniger angeregten Zustand wie seinen Grundzustand übergeht. Die Photolumineszenz des Leuchtelements, also die vom Leuchtelement ausgesandte elektromagnetische Strahlung, wird zur Beleuchtung des ophthalmischen Lichtinstruments verwendet. Das angeregte Leuchtelement befindet sich vorzugsweise in einem elektronisch angeregten Zustand und/oder in einem schwingungsangeregten Zustand. Oder anders gesagt ist ein angeregter Zustand des Leuchtelements vorzugsweise ein elektronisch angeregter und/oder schwingungsangeregter Zustand. Vorzugsweise befindet sich das angeregte Leuchtelement somit nicht im thermischen Gleichgewicht mit seiner Umgebung. Unter dem Grundzustand des Leuchtelements wird vorzugsweise ein Zustand von niedriger Energie verstanden als der angeregte Zustand. Insbesondere geht das angeregte Leuchtelement in Abwesenheit der Anregung durch die Anregungsquelle in den Grundzustand über. Im Grundzustand befindet sich das Leuchtelement vorzugsweise im thermischen Gleichgewicht mit seiner Umgebung. Unter der stimulierten Emission wird die Emission von elektromagnetischer Strahlung beziehungsweise Photonen verstanden, welche nicht spontan erfolgt, sondern durch andere Photonen ausgelöst wird, wodurch eine Kettenreaktion entsteht, in welcher angeregte Photonen, weitere Photonen anregen und somit zu einer stimulierten Emission von kohärentem Licht führt.

Vorzugsweise ist die Anregungsquelle ein Laser oder eine Laserdiode. Insbesondere handelt es sich bei der Anregungsquelle somit vorzugsweise um eine niedrig divergierende Leuchtquelle. Das Anregungslicht umfasst vorzugsweise mindestens einen Anregungsstrahl.

Eine bevorzugte Strahldivergenz des Anregungsstrahls hat beispielsweise eine Halbwertsbreite (Full Width at Half Maximum, FWHM) von 30° oder kleiner. Eine bevorzugte radiometrische Lichtleistung des Anregungsstrahls ist vorzugsweise 100 mW oder grösser.

Zusätzlich oder alternativ dazu weist der Anregungsstrahl der Anregungsquelle vorzugsweise beim Auftreffen auf dem Leuchtelement einen möglichst kleinen Radius auf. Je kleiner der Radius des Anregungsstrahls auf einer Fläche des Leuchtelements ist, desto höher ist die Leuchtdichte ($[lm/(sr*m^2)]$, da sich dieser angeregte Bereich des Leuchtelements von einem Zentrum des Anregungsstrahl nach aussen insbesondere gaussverteilt.

Eine Wellenlänge des Anregungslichts kann veränderbar sein. Oder anders gesagt kann es sich bei der Anregungsquelle um eine abstimmbare Anregungsquelle wie beispielsweise einen Laser handeln. Genauso denkbar ist jedoch, dass eine Wellenlänge des Anregungslichts unveränderbar ist, beispielsweise im Falle einer Anregungsquelle in Form einer Laserdiode. Unterschiedlichste Wellenlängen des Anregungslichts sind denkbar. Beispielsweise kann das Anregungslichts im UV-Bereich, insbesondere im UV-A Bereich, im nah-UV Bereich, violetten bis blauen Bereich des elektromagnetischen Spektrums liegen. Oder anders gesagt kann das Anregungslicht eine Wellenlänge im Bereich von etwa 380 Nanometer bis 460 Nanometer aufweisen. Zum Beispiel kann die Anregungsquelle eine Laserdiode sein, welche Anregungslicht einer Wellenlänge von 450 Nanometer oder 405 Nanometer oder 380 Nanometer emittiert. Das Leuchtelement weist ein Photolumineszenz-Spektrum auf, und wobei eine Wellenlänge des Anregungslichts vorzugsweise kleiner als eine Wellenlänge des Photolumineszenz-Spektrums ist.

Das Leuchtelement ist vorzugsweise derart ausgebildet, dass sein Photolumineszenz-Spektrum den sichtbaren Bereich des elektromagnetischen Spektrums, vorzugsweise ohne den violetten und tiefblauen Bereich des elektromagnetischen Spektrums, umfasst. Denkbare Wellenlängen des Photolumineszenz-Spektrums hängen vom Material des Leuchtelements und der Wellenlänge des Anregungslichts ab und liegen beispielsweise im Bereich von 420 Nanometer bis 780 Nanometer, z.B. im Bereich von 500 Nanometer bis 780 Nanometer. Beispiele von denkbaren Materialien sind $KEu(WO_4)_2$ mit einer maximalen Photolumineszenz-Intensität um 614 Nanometer herum, oder $BaMg_2Al_{16}O_{27}:Eu,Mn$ mit einer maximalen Photolumineszenz-Intensität um 515 Nanometer herum, oder $(Sr,Mg)_2SiO_4:Eu$ mit einer maximalen Photolumineszenz-Intensität um 460 Nanometer herum. Nochmals weitere Beispiele von denkbaren Leuchtelementen sind $Ca_3Sc_2Si_3O_{12}:Ce^{3+}$ mit einer Anregungswellenlänge von 450 Nanometer und einer maximalen Photolumineszenz-Intensität um 505 Nanometer herum, und/oder $HK_3SnF_8:Mn^{4+}$ mit einer Anregungswellenlänge von 472 Nanometer und einer maximalen Photolumineszenz-Intensität um 627 Nanometer herum, und/oder $Ca_3Sc2Si3O12:Ce^{3+},Mn_2^+$ mit einer Anregungswellenlänge von 444 Nanometer und einer maximalen Photolumineszenz-Intensität um 567 sowie 700 Nanometer herum, und/oder $K_2NbF_7:Mn^{4+}$ mit einer Anregungswellenlänge von 475 Nanometer und einer maximalen Photolumineszenz-Intensität um 627 Nanometer herum, und/oder $K_2TaF7:Mn4+$ mit einer Anregungswellenlänge von 473.5 Nanometer und einer maximalen Photolumineszenz-Intensität um 628 Nanometer herum, und/oder $Na_3GaF_6:Mn^{4+}$ mit einer Anregungswellenlänge von 415 Nanometer und einer maximalen Photolumineszenz-Intensität um 628 Nanometer herum, und/oder $ZnGeF_6:Mn^{4+}$ mit einer Anregungswellenlänge von 470 Nanometer und einer maximalen Photolumineszenz-Intensität um 629 Nanometer herum, und/oder $RbNa_3Mg_7(PO_4)_6:Eu$ mit einer Anregungswellenlänge von 380 Nanometer und einer maximalen Photolumineszenz-Intensität um 451 Nanometer herum. Es gilt zu verstehen, dass das Leuchtelement eine Vielzahl an weiteren Materialen umfassen kann, wobei diese dem Fachmann bekannt sind.

Die Anregungsquelle und/oder das Anregungslicht ist vorzugsweise in einem Winkel zum Leuchtelement, insbesondere zu einer Fläche des Leuchtelements, angeordnet. Der Winkel beträgt vorzugsweise etwa 30° oder mehr, beispielsweise 45° oder mehr wie etwa 90°. Oder anders gesagt ist die Anregungsquelle und/oder das Anregungslicht vorzugsweise in einem Winkel von etwa 30° bis 90° zu einer Fläche des Leuchtelements angeordnet. Nochmals anders gesagt ist die Anregungsquelle und/oder ihr Anregungslicht bzw. ein von ihr ausgesandter Anregungsstrahl derart zum Leuchtelement angeordnet sein, dass ein Winkel zwischen der Anregungsquelle bzw. dem Anregungslicht bzw. dem Anregungsstrahl und einer Flächennormalen auf die Fläche des Leuchtelements etwa 30° oder weniger, beispielsweise etwa 0° betragen. Ein Winkel von etwa 0° beispielsweise bedeutet, dass die Anregungsquelle bzw. deren Anregungslicht bzw. deren Anregungsstrahl senkrecht zur Fläche des Leuchtelements angeordnet bzw. gerichtet sind. Mit dem Winkel ändert sich eine Eindringtiefe des Anregungslichts in das Leuchtelement sowie eine Reflexion des Anregungslichts. Aus diesem Grund ist es bevorzugt, dass die Anregungsquelle und/oder das Anregungslicht nicht zu flach bezüglich der Fläche des Leuchtelements angeordnet sind. Beispielsweise erfolgt bei einem Winkel von weniger als 30° eine starke Reflexion des Anregungslichts des Leuchtelements. Bei diesen Flächen handelt es sich vorzugsweise um eine Frontfläche, welche insbesondere eine Frontseite des Leuchtelements umfasst oder bereitstellt, und eine Rückfläche, welche eine Rückseite des Leuchtelements umfasst oder bereitstellt, siehe nachfolgend.

Vorzugsweise weist das Leuchtelement also eine Frontseite und eine entgegengesetzte Rückseite auf. Das Leuchtelement ist vorzugsweise derart angeordnet, dass sich die Photolumineszenz ausgehend von der Frontseite des Leuchtelements entlang einer Ausbreitungsrichtung ausbreitet. Die Anregungsquelle kann bezüglich der Ausbreitungsrichtung gesehen vor dem Leuchtelement oder nach dem Leuchtelement angeordnet sein. Die Ausbreitungsrichtung der Photolumineszenz ist vorzugsweise parallel zu einer Flächennormalen des Leuchtelements und/oder senkrecht auf eine Fläche des Leuchtelements, siehe auch weiter unten. Die Anregungsquelle und/oder ihr Anregungslicht und/oder ein Anregungsstrahl der Anregungsquelle können der Frontseite und/oder der Rückseite des Leuchtelements zugeordnet sein. Die Anregungsquelle ist vorzugsweise ausserhalb der Ausbreitungsrichtung der Photolumineszenz angeordnet, da ansonsten die Photolumineszenz durch die Anregungsquelle blockiert würde. Falls die Anregungsquelle der Frontseite des Leuchtelements zugeordnet ist, oder anders gesagt bezüglich der Ausbreitungsrichtung gesehen nach dem Leuchtelement angeordnet ist, so ist die Anregungsquelle vorzugsweise bezüglich der Ausbreitungsrichtung gesehen seitlich versetzt vom Leuchtelement. Falls die Anregungsquelle der Rückseite des Leuchtelements zugeordnet ist, oder anders gesagt bezüglich der Ausbreitungsrichtung gesehen vor dem Leuchtelement angeordnet ist, kann die Anregungsquelle bezüglich der Ausbreitungsrichtung gesehen seitlich versetzt oder zumindest teilweise mit dem Leuchtelement überlagernd angeordnet sein. Ein bezüglich der Ausbreitungsrichtung zumindest teilweise mit dem Leuchtelement überlagernde Anregungsquelle könnte zum Beispiel in einem Winkel von etwa 90° bezüglich der Rückseite des Leuchtelements angeordnet sein, oder vorzugsweise derart, dass ein Winkel zwischen ihrem Anregungsstrahl und einer Flächennormalen des Leuchtelements etwa 0° beträgt. Bei dieser Überlagerung handelt es sich vorzugsweise nicht um eine physische oder strukturelle Überlagerung, bei welcher ein physischer Kontakt zwischen der Anregungsquelle und dem Leuchtelement ausgebildet wird. Stattdessen ist es bevorzugt, dass die Anregungsquelle beabstandet vom Leuchtelement angeordnet ist. Dies gilt vorzugsweise auch für den Fall, dass die Anregungsquelle der Frontseite des Leuchtelements zugewandt respektive bezüglich der Ausbreitungsrichtung gesehen nach dem Leuchtelement angeordnet ist. Das heisst, die ophthalmische Beleuchtungsvorrichtung gestattet eine Anregung des Leuchtelements unter verschiedenen Anregungsrichtungen wie frontseitig oder rückseitig auf das Leuchtelement oder unter verschiedenen Anregungswinkeln. Weiter kann genau eine Anregungsquelle oder zwei oder mehr Anregungsquellen vorgesehen sein. Aussagen betreffend eine Anregungsquelle treffen vorzugsweise analog auf zwei oder mehr Anregungsquellen zu oder umgekehrt. Im Falle von zwei Anregungsquellen sind diese vorzugsweise bezüglich der Ausbreitungsrichtung gesehen einander gegenüberliegend angeordnet. Zusätzlich oder alternativ dazu sind diese zwei Anregungsquellen bezüglich einer zentral durch das Leuchtelement verlaufenden Mittelachse einander gegenüberliegend angeordnet. Vorzugsweise verläuft die Ausbreitungsrichtung parallel zur Mittelachse. Beispielsweise kann eine erste Anregungsquelle bezüglich der Ausbreitungsrichtung oder Mittelachse gesehen links vom Leuchtelement und eine zweite Anregungsquelle bezüglich der Ausbreitungsrichtung oder Mittelachse gesehen rechts vom Leuchtelement angeordnet sein. Vorzugsweise sind diese zwei Anregungsquellen dabei in einem Winkel zum Leuchtelement, insbesondere zu einer Fläche des Leuchtelements wie der Frontfläche oder der Rückfläche, angeordnet. Der Winkel kann beispielsweise etwa 45° betragen. Zusätzlich oder alternativ dazu sind die zwei oder mehr Anregungsquellen bezüglich der Ausbreitungsrichtung gesehen nach dem Leuchtelement angeordnet.

Das Leuchtelement umfasst oder besteht vorzugsweise aus mindestens einem photolumineszierenden Material. Zusätzlich oder alternativ dazu ist das Leuchtelement vorzugsweise schichtförmig ausgebildet. Insbesondere umfasst oder besteht das Leuchtelement aus mindestens einer Schicht an photolumineszierendem Material. Beispielsweise kann es sich beim Leuchtelement um eine oder mehrere Schichten aus einem oder mehreren der obgenannten Materialien wie $KEu(WO_4)_2$, $BaMg_2Al_{16}O_{27}$:Eu,Mn oder $(Sr, Mg)_2SiO_4$:Eu handeln. Zusätzlich oder alternativ weist das Leuchtelement vorzugsweise eine Flächenausdehnung von etwa 1 Millimeter bis 5 Millimeter auf. Beispielsweise kann die Flächenausdehnung des Leuchtelements 5 Millimeter x 5 Millimeter, 2 Millimeter x 2 Millimeter, 1 Millimeter x 1 Millimeter oder weniger wie 0.75 Millimeter x 0.75 Millimeter sein. Anders gesagt ist das Leuchtelement vorzugsweise flächig, und insbesondere schichtförmig, ausgebildet, und erstreckt sich vorzugsweise entlang mindestens einer Ebene. Das flächig oder schichtförmig ausgebildete Leuchtelement weist vorzugsweise die obgenannten Flächen, insbesondere die Frontfläche und die Rückfläche, auf. Folglich ist eine Flächenausdehnung dieser Flächen vorzugsweise etwa 1 Millimeter bis 5 Millimeter. Andere Flächenausdehnungen sind jedoch genauso denkbar und im Hinblick auf eine Photolumineszenz-Intensität eher unkritisch, so hängt diese eher vom Lichtspot des Anregungslichts ab. Das liegt daran, dass sich die Photolumineszenz um den Lichtspot, also um einen Auftreffpunkt des Anregungslichts auf dem Leuchtelement verteilt.

Vorzugsweise sind die Anregungsquelle und das Leuchtelement derart angeordnet und ausgebildet, dass ein Kombinationsspektrum gebildet wird, das einerseits i) das Photolumineszenz-Spektrum des Leuchtelements und andererseits ii) durch das Leuchtelement transmittiertes Anregungslicht und/oder vom Leuchtelement reflektiertes Anregungslicht umfasst. Das Kombinationsspektrum umfasst oder besteht vorzugsweise aus dem transmittierten oder reflektierten Anregungslicht und der Photolumineszenz und bildet insbesondere bevorzugt Weisslicht. Das Beleuchtungslicht umfasst oder besteht vorzugsweise aus dem Weisslicht. Unter Weisslicht wird eine Mischung aus elektromagnetischer Strahlung verstanden, welche Wellenlängen im sichtbaren Bereich vom elektromagnetischen Spektrum aufweist. Das Kombinationsspektrum wird vorzugsweise durch diese Wellenlängen bereitgestellt. Das heisst, das Weisslicht kann sowohl die Photolumineszenz als auch transmittiertes und/oder reflektiertes Anregungslicht umfassen. Beispielsweise kann das Weisslicht aus blauem Anregungslicht mit einer Wellenlänge von 450 Nanometer einer blauen Laserdiode und gelbgrüner Photolumineszenz gebildet werden. Je nach Anordnung der Anregungsquelle in Bezug auf das Leuchtelement wird zumindest ein Teil des Anregungslichts vom Leuchtelement reflektiert respektive durch das Leuchtelement transmittiert, wobei ein Kombinationsspektrum gebildet wird, welches das blaue Licht der Laserdiode und das gelbgrüne Licht der Photolumineszenz und folglich Weisslicht umfasst. Oder anders gesagt wird durch Zusammensetzen der blauen und gelbgrünen Spektren Weisslicht gebildet.

Oder genereller gesagt können Anregungsquellen wie blaue Laserdioden mit Anregungslicht im Bereich von 440 Nanometer bis 490 Nanometer und ein Leuchtelement mit einer Photolumineszenz im Bereich von 480 Nanometer bis 700 Nanometer zur Erzeugung von Weisslicht verwendet werden. Andere Anregungsquellen bzw. Photolumineszenzen und folglich Kombinationsspektren sind natürlich genauso denkbar.

Vorzugsweise umfasst die ophthalmische Beleuchtungsvorrichtung mindestens ein Wärmeleitelement und mindestens ein Kühlelement. Das Wärmeleitelement ist vorzugsweise zum Leiten einer vom Anregungslicht erzeugten Wärme zum Kühlelement ausgebildet, und das Kühlelement ist vorzugsweise zum Abgeben der Wärme an die Umgebung ausgebildet. Das Wärmeleitelement ist vorzugsweise unmittelbar benachbart zum Leuchtelement angeordnet. Insbesondere bevorzugt ist das Wärmeleitelement in Flächenkontakt mit dem Leuchtelement, insbesondere mit der Rückfläche des Leuchtelements angeordnet. Zusätzlich oder alternativ dazu ist das Kühlelement vorzugsweise unmittelbar benachbart zum, insbesondere in Flächenkontakt mit dem Wärmeleitelement angeordnet. Entlang einer Ausdehnungsrichtung der ophthalmischen Beleuchtungsrichtung gesehen können das Leuchtelement gefolgt vom Wärmeleitelement gefolgt vom Kühlelement angeordnet sein. Die Ausdehnungsrichtung verläuft vorzugsweise parallel zur Dickenrichtung des Leuchtelements und/oder parallel zur Ausbreitungsrichtung der Photolumineszenz. Das Wärmeleitelement ist vorzugsweise aus einem wärmeleitenden Material, beispielsweise aus Kuper oder Aluminium, obwohl andere Materialien genauso denkbar sind. Beim Kühlelement kann es sich um einen Kühlkörper mit Lüfter handeln, welcher die Wärme an die Umgebungsluft abgeben kann. Weiter können das Leuchtelement, das Wärmeleitelement und das Kühlelement als einteiliges Bauelement bereitgestellt werden. Unter einteilig wird verstanden, dass diese Elemente nicht zerstörungsfrei voneinander getrennt werden können. Beispielsweise können diese Elemente stoffschlüssig miteinander verbunden sein, beispielsweise durch einen wärmeleitenden Kleber. Weiter ist es bevorzugt, dass ein thermischer Widerstand zwischen dem Wärmeleitelement und dem Kühlelement gering ist, z.B. durch Anordnung einer Paste oder einer Wärmeleitfolie wie einer Graphitfolie zwischen diesen Elementen. Vorzugsweise umfasst die ophthalmische Beleuchtungsvorrichtung mindestens ein Gehäuse, wobei das Leuchtelement und vorzugsweise das Wärmeleitelement und/oder das Kühlelement zumindest teilweise im Gehäuse angeordnet sind. Das heisst, das Leuchtelement, das Wärmeleitelement und das Kühlelement können zumindest teilweise und vorzugsweise vollständig innerhalb eines Gehäuses angeordnet sein.

Die ophthalmische Beleuchtungsvorrichtung umfasst vorzugsweise mindestens ein optisches Filterelement. Das optische Filterelement ist dazu ausgebildet, einfallendes Licht umfassend die Photolumineszenz und optional zumindest teilweise das Anregungslicht basierend auf mindestens einer optischen Eigenschaft des einfallenden Lichts, insbesondere der Wellenlänge, als Reflexionslicht zu reflektieren und als Transmissionslicht zu transmittieren, und wobei das Beleuchtungslicht das Reflexionslicht oder das Transmissionslicht umfasst oder daraus besteht. Zusätzlich oder alternativ dazu ist das optische Filterelement vorzugsweise dazu ausgebildet, das Anregungslicht zumindest teilweise aus dem Beleuchtungslicht zu filtern. Die ophthalmische Beleuchtungsvorrichtung umfasst also vorzugsweise mindestens ein optisches Filterelement, welches dazu ausgebildet ist, auf das optische Filterelement einfallendes Licht basierend auf mindestens einer optischen Eigenschaft des einfallenden Lichts zu reflektieren oder zu transmittieren. Bei der optischen Eigenschaft des einfallenden Lichts handelt es sich vorzugsweise um eine Wellenlänge des einfallenden Lichts. Eine Reflexion bzw. Transmission aufgrund anderer optischer Eigenschaften wie zum Beispiel basierend auf einer Polarisation des einfallenden Lichts und/oder basierend auf einem Einfallswinkel des einfallenden Lichts sind jedoch genauso denkbar. Insbesondere handelt es sich beim optischen Filterelement um einen Interferenzfilter wie beispielsweise um einen dichroitischen Spiegel, wie er allgemein bekannt ist. Das optische Filterelement hat also vorzugsweise für einfallendes Licht unterschiedlicher Wellenlängen und/oder unterschiedlichen Einfallswinkeln und/oder unterschiedlicher Polarisation einen verschiedenen Reflexions- und Transmissionsgrad. Abhängig vom einfallenden Licht und davon, ob das vom optischen Filterelement reflektierte oder transmittierte Licht verwendet wird, kann unterschiedliches Beleuchtungslicht erzeugt werden, beispielsweise Beleuchtungslicht unterschiedlich zusammengesetzter Spektren. Verständnishalber werden hierin das alsdann verwendete Licht als Ausgangslicht und das nicht verwendete Licht als Ausschusslicht bezeichnet. Das auf das optische Filterelement einfallende Licht umfasst vorzugsweise die vom Leuchtelement erzeugte Photolumineszenz. Vorzugsweise umfasst das einfallende Licht weiter zumindest teilweise das Anregungslicht. Insbesondere bevorzugt umfasst das einfallende Licht Anregungslicht, das vom Leuchtelement reflektiert wird und/oder durch das Leuchtelement transmittiert. Beispielsweise kann das einfallende Licht das zuvor beschriebene Weisslicht sowie das vom Leuchtelement reflektierte und/oder durch das Leuchtelement transmittierte Anregungslicht umfassen. In Kombination mit dem Filterelement ist es bevorzugt, dass die ophthalmische Beleuchtungsvorrichtung mindestens ein optisches Absorptionselement wie eine sogenannte Strahlfalle aufweist, welches das Ausschusslicht absorbiert. Andere Absorptionselemente sind jedoch genauso denkbar und dem Fachmann bekannt, wie beispielsweise ein schwarz matt eloxierter Linsentubus respektive ein Beamsplitter. Zusätzlich oder alternativ dazu ist das optische Filterelement vorzugsweise dazu ausgebildet ist, das Anregungslicht zumindest teilweise aus dem Beleuchtungslicht zu filtern. Anders gesagt kann das optische Filterelement zur Filterung respektive Abblockung des Anregungslichts ausgebildet sein, so dass das Anregungslicht nicht oder nur teilweise in das ophthalmische Lichtinstrument und somit ins Auge gestrahlt wird. Dadurch kann eine phototoxische Dosis verringert werden. In diesem Zusammenhang ist es bevorzugt, dass das optische Filterelement dazu ausgebildet ist, einfallendes Anregungslicht zu reflektieren und beispielsweise in ein optisches Absorptionselement wie eine sogenannte Strahlfalle zu leiten. Somit ist es bevorzugt, dass das optische Filterelement bei einer Wellenlänge des Anregungslichts eine hohe Reflektivität, beispielsweise von 95 % und grösser, insbesondere von grösser 99.5 % aufweist. Das optische Filterelement weist vorzugsweise eine Beschichtung auf, welche bei einem Peak, also bei einer zentralen Wellenlänge bzw. bei einer Wellenlänge von maximaler Intensität, des Anregungslichts diese hohe Reflektivität aufweist. Das optische Filterelement weist vorzugsweise eine "Cut-Off-Wellenlänge" auf, also eine Wellenlänge, bei der die Transmission auf 50 % abfällt. Dies gestattet gewissermassen eine selektive Auswahl an Wellenlängen, welche für das Beleuchtungslicht verwendet respektive aus diesem entfernt werden sollen. Das optische Filterelement ist vorzugsweise entlang der Ausbreitungsrichtung der Photolumineszenz gesehen nach dem Leuchtelement angeordnet. Weiter ist es bevorzugt, dass das optische Filterelement im Gehäuse angeordnet ist. Weiter ist es bevorzugt, dass das optische Filterelement bezüglich des Leuchtelements ausgerichtet respektive entlang der Ausbreitungsrichtung der Photolumineszenz angeordnet ist. Wie nachfolgend noch eingehender erläutert wird, definiert die ophthalmische Beleuchtungsvorrichtung, insbesondere eine Kollektorlinse zur parallelen Ausrichtung der divergenten Photolumineszenz (siehe weiter unten), vorzugsweise eine optische Achse. Die optische Achse ist vorzugsweise parallel zur Ausbreitungsrichtung der Photolumineszenz und/oder parallel zu einer Flächennormalen, insbesondere zur Flächennormalen auf die Frontfläche, des Leuchtelements und/oder parallel zur Mittelachse durch das Leuchtelement. Das optische Filterelement ist vorzugsweise auf der optischen Achse angeordnet.

Die ophthalmische Beleuchtungsvorrichtung umfasst vorzugsweise mindestens eine ergänzende Leuchtquelle zur Aussendung von Ergänzungslicht auf das optische Filterelement derart, dass ein ergänztes Kombinationsspektrum gebildet wird, welches einerseits i) das Reflexionslicht oder das Transmissionslicht und andererseits ii) das Ergänzungslicht umfasst. In diesem Fall umfasst oder besteht das Beleuchtungslicht vorzugsweise aus dem ergänzten Kombinationsspektrum.

Vorzugsweise handelt es sich beim auf das optische Filterelement einfallenden Licht somit um die Photolumineszenz des Leuchtelements, um das Ergänzungslicht der ergänzenden Leuchtquelle sowie optional weiter um zumindest einen Teil des Anregungslichts, welches von Leuchtelement reflektiert und/oder durch das Leuchtelement transmittiert wurde. Entsprechend kann das optische Filterelement Reflexionslicht und/oder Transmissionslicht erzeugen, welches die Photolumineszenz und das Ergänzungslicht sowie optional zumindest einen Teil des Anregungslichts umfasst, und wobei das Beleuchtungslicht dieses Reflexionslicht oder Transmissionslicht umfasst oder daraus besteht. Vorzugsweise ist die ergänzende Leuchtquelle derart angeordnet, dass das Ergänzungslicht entlang einer Ausbreitungsrichtung verläuft, welche senkrecht zur Ausbreitungsrichtung der Photolumineszenz und/oder senkrecht zur optischen Achse verläuft. Eine Wellenlänge des Ergänzungslichts kann veränderbar oder unveränderbar sein. Zudem können unterschiedliche ergänzende Leuchtquellen mit unterschiedlichen Wellenlängen verwendet werden. Bei der ergänzenden Leuchtquelle kann es sich um eine abstimmbare Leuchtquelle, beispielsweise um einen Laser, oder um eine Laserdiode oder eine LED handeln. Die Wellenlänge des Ergänzungslichts liegt vorzugsweise im sichtbaren Bereich des elektromagnetischen Spektrums. Insbesondere ist die Wellenlänge des Ergänzungslichts derart, dass sich ein Kombinationsspektrum aus Weisslicht ergibt. Beispielsweise kann als Anregungsquelle eine blaue Laserdiode, ein grün photolumineszierendes Leuchtelement und rotes Ergänzungslicht von einer roten Laserdiode zur Erzeugung von Weisslicht verwendet werden. Das heisst, eine Wellenlänge des Ergänzungslichts kann gleich wie oder verschieden von einer Wellenlänge des Anregungslichts sein. Beispielsweise kann es sich bei der Anregungsquelle sowie bei der ergänzenden Leuchtquelle jeweils um eine blaue Laserdiode handeln, welche Ergänzungslicht bzw. Anregungslicht in Form von blauer Laserstrahlung respektive mit einer Wellenlänge von 450 Nanometer emittiert. Genauso denkbar ist es jedoch, dass sich eine Wellenlänge des Ergänzungslichts von einer Wellenlänge des Anregungslichts unterscheidet. Beispielsweise kann es sich bei der Anregungsquelle um die soeben genannte blauer Laserdiode mit Wellenlänge von 450 Nanometer handeln, während die ergänzende Leuchtquelle eine Laserdiode von anderer Farbe beziehungsweise Wellenlänge ist, z.B. eine grüne Laserdiode mit Wellenlänge 514 Nanometer oder eine rote Laserdiode mit Wellenlänge 630 Nanometer. Das Vorsehen von mindestens einer ergänzenden Leuchtquelle sowie des optischen Filterelements gestattet eine für die Ophthalmologie optimale Einstellung der Farbspektren des Beleuchtungslichts. Aufgrund der Lichtbrechung und Streuung eignet sich beim Beleuchtungslicht die Farbe Blau zur Hervorhebung der Filamente im Glaskörper, wohingegen gelbrotes Licht zur Hervorhebung der Netzhaut vorteilhaft ist. Zurückkommend auf obiges Beispiel umfassend die ergänzende Leuchtquelle könnte nun also blaues Ergänzungslicht einer blauen Laserdiode auf das optische Filterelement in Form eines dichroitischen Spiegels gestrahlt werden. Wie erwähnt reflektiert respektive transmittiert der dichroitische Spiegel das einfallende Licht in Abhängigkeit von dessen Wellenlänge. Weiter könnte Weisslicht aus Photolumineszenz des Leuchtelements und reflektiertem Anregungslicht der Anregungsquelle ebenfalls auf den dichroitischen Spiegel gestrahlt werden, wobei dieser vorzugsweise derart ausgebildet ist, dass das Anregungslicht der Anregungsquelle aufgrund der Wellenlänge am dichroitischen Spiegel reflektiert und in eine Strahlfalle geführt und nicht transmittiert wird. Je nach Einstellwert und Bestromung der Laserdioden kann somit blaues, weisses oder gelbes Beleuchtungslicht erzeugt werden.

Das heisst, und wie nachfolgend noch eingehender erläutert wird, gestattet die ophthalmologische Beleuchtungsvorrichtung eine Farbänderung respektive Farbeinstellbarkeit, also eine Änderung der Zusammensetzung der Farbspektren, des Beleuchtungslichts basierend auf einer Leistungsregelung der Anregungsquelle bzw. der Leuchtquellen. Es gilt zu verstehen, dass ergänzende Leuchtquellen von anderer Wellenlänge genauso verwendet werden können. Beispielsweise könnte eine grüne oder roten Laserdiode anstelle der blauen Laserdiode verwendet werden, was eine Farbeinstellbarkeit der ophthalmischen Beleuchtungsvorrichtung von gelb bis grün respektive von gelb bis rot gestattet. Weiter ist es denkbar, dass die ophthalmische Beleuchtungsvorrichtung zwei oder mehr optische Filterelemente und/oder zwei oder mehr ergänzende Leuchtquellen umfasst. Die zwei oder mehr optischen Filterelemente sind vorzugsweise entlang der Ausbreitungsrichtung der Photolumineszenz und/oder auf der optischen Achse und insbesondere aufeinanderfolgend angeordnet. Die zwei oder mehr ergänzenden Leuchtquellen sind vorzugsweise derart angeordnet, dass deren jeweiliges Ergänzungslicht entlang jeweils einer Ausbreitungsrichtung verläuft, welche senkrecht zur Ausbreitungsrichtung der Photolumineszenz und/oder senkrecht zur optischen Achse verläuft. Weiter ist es bevorzugt, dass jeweils ein optisches Filterelement einer ergänzenden Leuchtquelle zugeordnet ist. Oder anders gesagt sind jeweils ein optisches Filterelement und eine ergänzende Leuchtquelle derart angeordnet, dass das Ergänzungslicht der besagten ergänzenden Leuchtquelle auf das besagte optische Filterelement einfällt. Weiter ist es bevorzugt, dass das einfallende Licht auf ein nachfolgendes optisches Filterelement jeweils Reflexionslicht oder Transmissionslicht von einem vorhergehenden optischen Filterelement umfasst. Eine Wellenlänge von zwei oder mehr ergänzenden Leuchtquellen kann dieselbe oder voneinander verschieden sein.

Das heisst, die ophthalmische Beleuchtungsvorrichtung kann mehrere ergänzende Leuchtquellen aufweisen, so dass beispielsweise nebst einer ersten ergänzenden Leuchtquelle in Form einer blauen Laserdiode und einem zugehörigen ersten optischen Filterelement wie einem ersten dichroitischen Spiegel eine zweite ergänzende Leuchtquelle in Form einer roten Laserdiode vorgesehen ist. Die Abstrahlung der roten Laserdiode kann auf ein zweites optisches Filterelement wie einen zur roten Laserdiode zugehörigen dichroitischen Spiegel gestrahlt werden. Dieses zweite optische Filterelement weist vorzugsweise eine höhere Cut-Off-Wellenlänge auf, sodass Wellenlängen von kleiner als 650-700 Nanometer (blau - gelb) durch das zweite optische Filterelement transmittiert und Wellenlängen von grösser als 650-700 Nanometer reflektiert werden. Dies erweitert den einstellbaren Farbraum für einen Benutzer der ophthalmischen Beleuchtungsvorrichtung. Das Vorsehen von einer oder mehreren ergänzenden Leuchtquellen führt zu einer Minimierung oder sogar Verschwinden von sogenannten Farbringen und gestattet eine farbhomogene Ausleuchtung des ophthalmischen Lichtinstruments und dadurch Auges mit einer linearen Farbregelung.

Das aus der ophthalmischen Beleuchtungsvorrichtung abgestrahlte Beleuchtungslicht bildet vorzugsweise einen objektseitigen Abstrahlwinkel $\varphi$ und das durch die ophthalmische Beleuchtungsvorrichtung zu beleuchtende ophthalmische Lichtinstrument definiert vorzugsweise einen bildseitigen Abstrahlwinkel $\varphi'$.

Die ophthalmische Beleuchtungsvorrichtung ist vorzugsweise derart ausgebildet, dass ein Abbildungsmassstab M der ophthalmischen Beleuchtungsvorrichtung einen Referenz-Abbildungsmassstab nicht überschreitet und/oder unterschreitet.

Es gilt:

$$M = \frac{\varphi}{\varphi'} = \frac{y'}{y}$$

mit $\varphi$ bzw. $\varphi'$: objekt- bzw. bildseitiger Abstrahlwinkel, und

mit y'= Bildgrösse (= z.B. Spot auf Faser des Lichtinstruments), und

mit y = Objektgrösse (= Ausdehnung auf dem Leuchtelement). Aus dieser Beziehung geht hervor, dass sich Winkel und Objektgrösse genau gegensätzlich verhalten. Wird also die Bildgrösse verkleinert (M<1), vergrössert man gleichzeitig den bildseitigen Winkel.

Zusätzlich oder alternativ dazu ist die ophthalmische Beleuchtungsvorrichtung vorzugsweise derart ausgebildet, dass der bildseitige Abstrahlwinkel des ophthalmischen Lichtinstruments nicht überschritten und/oder unterschritten wird.

Wie nachfolgend eingehender erörtert wird, umfasst das ophthalmische Lichtinstrument vorzugsweise eine Faser. Die Faser definiert eine Numerische Apertur NA, welche mit dem bildseitigen Abstrahlwinkel und folglich mit dem Abbildungsmassstab zusammenhängt.

Eine typische Numerische Apertur im Gebiet der Ophthalmologie beispielsweise ist 0.5, was einem bildseitigen Abstrahlwinkel von 60° entspricht. Ein objektseitiger Abstrahlwinkel der ophthalmischen Beleuchtungsvorrichtung, insbesondere der Anregungsquelle und, falls vorhanden, der ergänzenden Leuchtquelle, sei beispielsweise 120° FWHM, so dass sich ein Abbildungsmassstab von 2 ergibt. Geht man bspw. von einer Leuchtquelle in Form einer LED mit Objektgrösse 1x1 mm (Chipfläche) aus so wäre die Bildgrösse 2x2 mm. Kleine Fasern mit einem Durchmesser <300 $\mu$m würden also deutlich überstrahlt werden und der einkoppelbare Lichtstrom sinkt. Die ophthalmische Beleuchtungsvorrichtung weist daher einen Abbildungsmassstab auf, welcher einen optimalen Lichtstrom und eine optimale Einkopplungseffizienz des Beleuchtungslichts in das ophthalmische Lichtinstrument bewirkt. Die Numerische Apertur der Faser NA kann aus dem Produkt des Sinus eines halben Öffnungswinkels und dem der Faser gegenüber Luft berechnet werden. Der Öffnungswinkel wiederum entspricht dem Austrittswinkel einer Quelle oder einer Faser, hier also - 120° FWHM voller Öffnungswinkel.

So wird M definiert durch die NA der Faser eines ophthalmischen Lichtinstruments und der Lichtstärkeverteilungskurve (gaussverteilt) der Leuchtquelle (beispielsweise also LED oder Photolumineszenz). Daraus kann für unterschiedliche Bildgrössen (hier also Faserdurchmesser) der einkoppelbare Lichtstrom für unterschiedliche Abbildungsmassstäbe berechnet werden. Wenn nun die NA einer Faser, der Durchmesser der Faser und die Lichtstärkeverteilungskurve der Leuchtquelle bekannt sind, kann derjenige Abbildungsmassstab ermittelt werden, bei dem sich der meiste Lichtstrom in die gegebene Fasergrössen einkoppeln lässt.

Die ophthalmische Beleuchtungsvorrichtung umfasst vorzugsweise eine Kollektoroptik und eine Kondensoroptik, welche jeweils mindestens eine Linse umfasst. Die Kollektoroptik ist derart angeordnet und ausgebildet, dass sie die Strahlrichtung der Photolumineszenz des Leuchtelements zumindest teilweise entlang einer optischen Achse ausrichtet. Die Kondensorlinse ist derart angeordnet und ausgebildet, dass sie das Beleuchtungslicht aus der Beleuchtungsvorrichtung in ein ophthalmisches Lichtinstrument ausgibt. Vorzugsweise sind die Kollektoroptik und die Kondensoroptik jeweils als achromatisches Linsenpaar realisiert. Unter einem achromatisches Linsenpaar wird die Kombination von Linsen mit unterschiedlichem Dispersionsverhalten (Abbe-Zahl) verstanden. Die Verwendung von achromatischen Linsenpaaren gestattet eine Minimierung der chromatischen Aberration. Das heisst, die Kollektoroptik und die Kondensoroptik werden jeweils durch achromatische Linsenpaare realisiert, wodurch eine chromatische Aberration aufgrund unterschiedlicher Wellenlängen beispielsweise der Photolumineszenz und/oder dem Anregungslicht und/oder dem Ergänzungslicht minimiert wird. Somit stellen die achromatischen Linsenpaare der Kollektoroptik die Kollektor-Gruppe dar und die achromatischen Linsenpaare der Kondensoroptik stellen die Kondensor-Gruppe dar.

Zusätzlich oder alternativ dazu kann die ophthalmische Beleuchtungsvorrichtung optische Elemente, insbesondere Linsen oder Linsensysteme umfassen. Beispielsweise kann sie ein Linsensystem umfassend eine Kollimationslinse und eine Fokuslinse umfassen, wobei die Kollimationslinse dazu dient, die Photolumineszenz und optional zumindest teilweise das Anregungslicht zunächst zu kollimieren und alsdann das erzeugte Beleuchtungslicht mittels der Fokuslinse auf das Lichtinstrument, insbesondere auf eine Fasereinkopplung des Lichtinstruments zu fokussieren. Zusätzlich oder alternativ dazu kann sie eine der ergänzenden Leuchtquelle zugeordnete Linse, insbesondere eine Kollimationslinse aufweisen, welche einer Kollimation des Ergänzungslicht dient. Sind mehrere ergänzende Leuchtquellen vorhanden, so sind vorzugsweise mehrere Kollimationslinsen vorhanden.

Die ophthalmische Beleuchtungsvorrichtung umfasst vorzugsweise mindestens ein Sensorelement zur Erfassung einer Strahlungsleistung des Beleuchtungslichts und/oder des Anregungslichts und/oder des Ergänzungslichts. Das Sensorelement ist vorzugsweise teilweise in einem Strahlengang des Beleuchtungslichts und/oder des Anregungslichts und/oder des Ergänzungslichts angeordnet. Die ophthalmische Beleuchtungsvorrichtung ist vorzugsweise dazu ausgebildet, ein auf der erfassten Strahlungsleistung basiertes Regelsignal an die Anregungsquelle und/oder die ergänzende Leuchtquelle zur Leistungsregelung der Anregungsquelle und/oder der ergänzenden Leuchtquelle zu senden. Das heisst, die ophthalmische Beleuchtungsvorrichtung umfasst vorzugsweise mindestens ein Sensorelement, welches teilweise im Strahlengang von mindestens einer und vorzugsweise von allen Leuchtquellen der ophthalmischen Beleuchtungsvorrichtung angeordnet ist. Vorzugsweise ist also mindestens ein Sensorelement teilweise im Strahlengang der Anregungsquelle, also im Anregungslicht, und/oder im Strahlengang der mindestens einer ergänzenden Leuchtquelle, also im mindestens einen Ergänzungslicht, und/oder im Strahlengang des Beleuchtungslichts angeordnet. Der Strahlengang des Beleuchtungslichts umfasst vorzugsweise den Strahlengang des Weisslichts und/oder des Reflexionslichts und/oder des Transmissionslichts.

Vorzugsweise ist das Sensorelement dabei jeweils im Randbereich des Strahlengangs angeordnet. Unter dem Randbereich des Strahlengangs wird vorzugsweise ein bezüglich einer radialen Richtung des jeweiligen Lichtstrahls gesehen äusserer Bereich des Lichtstrahls und/oder ein Bereich von geringerer Strahlungsintensität verstanden. Anders gesagt sind die Lichtstrahlen, also das Anregungslicht und/oder Ergänzungslicht und/oder Beleuchtungslicht, vorzugsweise gaussförmig und verfügen über eine gaussförmige Leistungsverteilung, und wobei das Sensorelement vorzugsweise in Intensitätsbereichen kleiner als 1/e der Apertur angeordnet ist. Nochmals anders gesagt ist das Sensorelement vorzugsweise im äusseren Bereich der Apertur angeordnet. Insbesondere können den Lichtstrahlen, also dem Anregungslicht und/oder Ergänzungslicht und/oder Beleuchtungslicht jeweils eine volle Apertur zugeordnet werden, wobei die volle Apertur dem effektiven Strahldurchmesser eines gaussverteilten Strahls entspricht. Die volle Apertur ist gekennzeichnet durch den Strahlradius, bei dem die optische Intensität auf $1/e^2$ (circa 13.53 %) des Intensitätsmaximums, welches im Mittelpunkt des effektiven Strahldurchmessers respektive entlang der optischen Achse liegt, abfällt. Das heisst, der Mittelpunkt des effektiven Strahldurchmessers ist gekennzeichnet durch das Intensitätsmaximums des gaussverteilten Strahlengangs und liegt auf der optischen Achse. Der Strahldurchmesser ohne Abschattung weist einen Strahlenradius auf, bei dem die optische Intensität auf 60-70 % des Intensitätsmaximums, welcher im Mittelpunkt respektive entlang der optischen Achse liegt, abgefallen ist. Der Randbereich des Strahlengangs, in dem das Sensorelement jeweils vorzugsweise angeordnet ist, liegt vorzugsweise im Querschnitt des Strahlengangs gesehen vorzugsweise zwischen einem Mindest-Strahlradius, bei dem die Intensität auf mindestens 60% und maximal 70% des Intensitätsmaximums abfällt, und der vollen Apertur respektive dem vollen Strahlenradius, bei dem die Intensität auf 13.5% ($1/e^2$) des Intensitätsmaximums abgefallen ist. So entspricht der volle Strahlenradius 13.5% des Intensitätsmaximums, folglich ist der Querschnitt (die Ringfläche) mindestens der Bereich zwischen 13.5 % und 60% des Intensitätsmaximums und maximal der Bereich zwischen 13.5% und 70% des Intensitätsmaximums. Anders als bei einer indirekten Erfassung der Strahlungsleistung beispielsweise mittels Messung einer Teilreflexion wird ist hier also das Sensorelement abschattend angeordnet und erfasst die Strahlungsleistung direkt im Strahlengang. Die Anordnung des Sensorelements im Randbereich hat dabei zur Folge, dass nur ein kleiner Teil der Leistung durch das Sensorelement abgeschattet wird. Wie nachfolgend noch eingehender erörtert wird, wird mit dem Beleuchtungslicht der ophthalmischen Beleuchtungsvorrichtung vorzugsweise ein ophthalmisches Lichtinstrument beleuchtet, welches über einen Lichtleiter beispielsweise in Form einer Faser verfügen kann. In diesem Fall kann eine Abschattung durch das bzw. die Sensorelemente durch eine Totalreflexion des Beleuchtungslichts innerhalb der Faser kompensiert werden. Insbesondere erfolgt eine Vielzahl von Totalreflexionen, was zu einer "Durchmischung" des Lichts führt, wodurch die Abschattung am Ausgang der Faser nicht mehr sichtbar ist. Zudem ermöglicht die Anordnung der Sensorelemente im Strahlengang der Leuchtquellen, dass der effektiv emittierte Lichtstrom der Leuchtquellen gemessen werden kann. Dadurch können Störgrössen wie die Degradierung des Lichtstroms über die Zeit oder die Ungenauigkeit durch Temperaturschwankungen eliminiert werden.

Beim Sensorelement kann es sich um eine Photodiode handeln, obwohl andere optische Sensorelemente genauso denkbar sind wie z.B. Photowiderstände, Phototransistoren, Arrays, etc.

Vorzugsweise ist die ophthalmische Beleuchtungsvorrichtung dazu ausgebildet, basierend auf der vom Sensorelement erfassten jeweiligen Strahlungsleistung ein Regelsignal zu ermitteln und dieses Regelsignal an die jeweilige Leuchtquelle, insbesondere also an die Anregungsquelle und/oder die mindestens eine ergänzende Leuchtquelle, zur Leistungsregelung der jeweiligen Leuchtquelle zu senden. Vorzugsweise umfasst die ophthalmische Beleuchtungsvorrichtung dazu mindestens eine Regelungsschaltung. Die Regelungsschaltung ist vorzugsweise mit dem Sensorelement in Verbindung und dazu ausgebildet, die vom Sensorelement erfasste Strahlungsleistung zu erhalten und darauf basierend ein Regelsignal zu ermitteln. Beispielsweise kann die Regelungsschaltung dazu ausgebildet sein, das Regelsignal basierend auf einem Vergleich der erfassten Strahlungsleistung mit Referenz-Strahlungsleistungen zu ermitteln. Insofern ist es bevorzugt, dass Referenz-Strahlungsleistungen in einem Speicher der Regelungsschaltung abgespeichert sind. Zusätzlich oder alternativ dazu ist es denkbar, dass die Regelungsschaltung dazu ausgebildet ist, Referenz-Strahlungsleistungen von einem entfernten Netzstandort aus über ein beliebiges geeignetes Dateiübertragungsprotokoll zu erhalten.

Weiter ist die Regelungsschaltung vorzugsweise mit der bzw. den Leuchtquellen in Verbindung und dazu ausgebildet, das ermittelte Regelsignal an die jeweilige Leuchtquelle, insbesondere an eine Steuergrösse der Leuchtquelle zu übermitteln, welche daraufhin die Leistung des von ihr ausgesandten Lichts entsprechend regelt.

Beispielsweise kann ein Sensorelement in Form einer Photodiode im Strahlengang einer ergänzenden Leuchtquelle in Form einer Laserdiode angeordnet sein und die Strahlungsleistung der Laserdiode messen. Die Photodiode erzeugt in Abhängigkeit der gemessenen Strahlungsleistung ein Stromsignal, welches an die Regelungsschaltung übermittelt wird und welche darauf basierend ein Regelsignal in Form eines Stromsignals erzeugt, welches zur Laserdiode zurückgeführt wird.

Insbesondere gestattet diese Regelung dabei die zuvor bereits erwähnte Farbänderung respektive Farbeinstellbarkeit des Beleuchtungslichts. Das heisst, bei der Leistungsregelung wird vorzugsweise die Leistung beziehungsweise die Bestromung der Leuchtquellen wie der Laserdioden geregelt, welche als Folge davon heller oder dunkler erscheint und einer Farbänderung respektive Farbeinstellbarkeit entspricht. Das heisst, die Leuchtquellen selbst müssen nicht abstimmbar sein, aber durch unterschiedliche Leistungsverhältnisse zueinander können sich in der Mischung bzw. dem Kombinationsspektrum unterschiedliche Farben des Beleuchtungslichts ergeben. Beispielsweise kann die ophthalmische Beleuchtungsvorrichtung eine Anregungsquelle in Form einer blauen Laserdiode, ein gelb photolumineszierendes Leuchtelement und eine ergänzende Leuchtquelle in Form einer blauen Laserdiode umfassen, und wobei

- bei einer Leistung der anregenden Laserdiode von 100% und bei einer Leistung der ergänzenden Leuchtquelle von 10% sehr gelbes Licht Beleuchtungslicht erzeugt wird; oder
- bei einer Leistung der anregenden Laserdiode von 100% und bei einer Leistung der ergänzenden Leuchtquelle von 100% Beleuchtungslicht in Form von Weisslicht erzeugt wird; oder
- bei einer Leistung der anregenden Laserdiode von 0% und bei einer Leistung der ergänzenden Leuchtquelle von 100% rein blaues Licht erzeugt wird;
- etc.

Nebst der Strahlungsleistung können auch weitere Grössen wie Störgrössen, z.B. eine Temperatur des Sensorelements oder eine spektrale Empfindlichkeit des Sensorelements erfasst und bei der Leistungsregelung mitberücksichtigt werden.

Weiter gestattet diese Leistungsregelung eine Angabe der phototoxischen Dosis in Abhängigkeit von dem tatsächlich emittierten Lichtstrom der Leuchtquelle. Das heisst, und wie nachfolgend noch eingehender erläutert wird, kann die ophthalmische Beleuchtungsvorrichtung mindestens ein Erkennungselement zum Erkennen des jeweils angeschlossenen Lichtinstrumentyps aufweisen. Aufgrund des soeben beschriebenen Regelkreises für das jeweils angeschlossene Lichtinstrument kann der einzukoppelnde Lichtstrom gemessen werden, was eine genaue Berechnung der phototoxischen Dosis ermöglicht. Letztere kann aus der von der ophthalmischen Beleuchtungsvorrichtung respektive vom Lichtinstrument emittierten Lichtleistung über die Zeit (= Lichtenergie) multipliziert mit dem jeweiligen Effizienzwert der Gauge-Grösse des Lichtinstrumentes ermittelt werden. Die Gauge-Grösse des Lichtinstruments steht mit dem Durchmesser des Lichtinstruments, insbesondere von deren Faser, in Verbindung. Allgemein gilt: Je kleiner die Gauge-Grösse, desto grösser ist der Durchmesser der Faser. Zum Beispiel hat eine 20-Gauge-Faser einen grösseren Durchmesser als eine 25-Gauge-Faser. Die Durchmesser je Gauge-Grösse sind in der EN ISO 9626:2016 definiert.

Vorzugsweise umfasst die ophthalmische Beleuchtungsvorrichtung mindestens ein Erkennungselement zum Erkennen eines erkennbaren Elements eines mit der ophthalmischen Beleuchtungsvorrichtung verbundenen ophthalmischen Lichtinstruments. Die ophthalmische Beleuchtungsvorrichtung ist vorzugsweise dazu ausgebildet, ein auf dem erkannten erkennbaren Element basiertes Regelsignal an die Anregungsquelle und/oder die ergänzende Leuchtquelle zur Leistungsregelung der Anregungsquelle und/oder der ergänzenden Leuchtquelle zu senden, und wobei das erkennbare Element vorzugsweise auf einem Instrumententyp des ophthalmischen Lichtinstruments basiert. Das heisst, vorzugsweise ist die ophthalmische Beleuchtungsvorrichtung dazu ausgebildet, basierend auf dem vom Erkennungselement erkannten erkennbaren Element wie dem Instrumententyp des ophthalmischen Lichtinstruments ein Regelsignal zu ermitteln und dieses Regelsignal an die jeweilige Leuchtquelle, insbesondere also an die Anregungsquelle und/oder die mindestens eine ergänzende Leuchtquelle, zur Leistungsregelung der jeweiligen Leuchtquelle zu senden. Insbesondere umfasst der Instrumententyp eine Fasergrösse des ophthalmischen Lichtinstruments. Je nachdem welche Faser an die ophthalmische Beleuchtungsvorrichtung angeschlossen wird, kann die ophthalmische Beleuchtungsvorrichtung mehr oder weniger Beleuchtungslicht in die Faser einkoppeln. Vorzugsweise umfasst die ophthalmische Beleuchtungsvorrichtung dazu mindestens eine Regelungsschaltung. Insbesondere bevorzugt handelt es sich dabei um die zuvor genannte Regelungsschaltung, welche mit dem mindestens einen Sensorelement in Verbindung steht. Die Regelungsschaltung ist vorzugsweise mit dem Erkennungselement in Verbindung und dazu ausgebildet, den vom Erkennungselement erkannten Instrumententyp zu erhalten und darauf basierend ein Regelsignal zu ermitteln. Beispielsweise kann die Regelungsschaltung dazu ausgebildet sein, das Regelsignal basierend auf einem Vergleich des erkannten Instrumententyps mit Referenz-Instrumententypen zu ermitteln. Insofern ist es bevorzugt, dass Referenz-Instrumententypen in einem Speicher der Regelungsschaltung abgespeichert sind. Zusätzlich oder alternativ dazu ist es denkbar, dass die Regelungsschaltung dazu ausgebildet ist, Referenz-Instrumententypen von einem entfernten Netzstandort aus über ein beliebiges geeignetes Dateiübertragungsprotokoll zu erhalten. Weiter ist die Regelungsschaltung vorzugsweise mit der bzw. den Leuchtquellen in Verbindung und dazu ausgebildet, das ermittelte Regelsignal an die jeweilige Leuchtquelle, insbesondere an eine Steuergrösse der Leuchtquelle zu übermitteln, welche daraufhin die Leistung des von ihr ausgesandten Lichts entsprechend regelt.

Verschiedenste Erkennungselemente sind denkbar wie zum Beispiel eine RFID-Antenne zur Erkennung eines am ophthalmischen Lichtinstrument angeordneten RFID-Tags, ein QR-Code Lesegerät zum Lesen eines am ophthalmischen Lichtinstrument angeordneten QR-Codes, etc. Das heisst, vorzugsweise umfasst das mit der ophthalmischen Beleuchtungsvorrichtung anzuschliessende ophthalmische Lichtinstrument mindestens ein erkennbares Element, welches vom Erkennungselement der ophthalmischen Beleuchtungsvorrichtung erkannt werden kann. Verschiedenste Anordnungen des Erkennungselements an der ophthalmischen Beleuchtungsvorrichtung sind denkbar, solange eine Erkennung des angeschlossenen ophthalmischen Lichtinstruments möglich ist und eine Beleuchtung des ophthalmischen Lichtinstruments nicht gestört wird.

Beispielsweise kann das Erkennungselement an einer Buchse der ophthalmischen Beleuchtungsvorrichtung angeordnet sein, und das erkennbare Element kann an einem Stecker des ophthalmischen Lichtinstruments angeordnet sein, wobei der Stecker an die Buchse und folglich das ophthalmische Lichtinstrument an die ophthalmische Beleuchtungsvorrichtung angeschlossen wird. Das Erkennungselement kann zum Erkennen von verschiedensten Instrumententypen ausgebildet sein. Beispielsweise kann es sich bei den Instrumententypen um ophthalmische Lichtinstrumente verschiedener Instrumentengrösse wie zum Beispiel verschiedene Gauge-Grössen oder Instrumentenarten wie z.B. Panorama, Chandeliers, etc. handeln.

Durch Verwendung von Anregungslicht in Form von stimulierter Emission und optional zusätzlich von ergänzenden Leuchtquellen wie eine leistungsstarke Laserdiode in ophthalmische Lichtinstrumente umfassend beispielsweise grössere Lichtfasern (beispielsweise mit einem Faserdurchmesser ø $\geq$400 $\mu$m) kann derart viel Lichtstrom eingekoppelt werden, dass thermische Verformungen der Lichtfasern entstehen können. Aus diesem Grund ist es von Vorteil, dass der Instrumententyp, insbesondere eine Gauge-Grösse des ophthalmischen Lichtinstrumentes erkannt und der Lichtstrom der Leuchtquelle entsprechend geregelt wird. Zur Erkennung kann auf dem ophthalmischen Lichtinstrument ein erkennbares Element beispielsweise in Form eines RFID-Tag angebracht werden. Dieser wird durch ein Erkennungselement beispielswese in Form einer RFID-Leserantenne auf der Buchse der ophthalmischen Beleuchtungsvorrichtung angeregt. Das so entstandene Signal kann drahtgebunden, z.B. über ein Koaxialkabel an eine Steuerelektronik oder Steuerprint wie einen RFID-Leser übertragen und dort ausgewertet werden. Abhängig von der Fasergrösse wird der einkoppelbare Lichtstrom, also der Lichtstrom am Ort der Fasereinkopplung begrenzt. Dadurch stehen für den Anwender für die Gauge-Grössen 20G, 23G, 25G und 27G identische maximale Lichtstromwerte am Faserausgang zur Verfügung.

Insbesondere ist es bevorzugt, dass eine Leistungsregelung basierend auf sowohl der vom Sensorelement erfassen Strahlungsleistung des Beleuchtungslichts bzw. des Anregungslichts bzw. des Ergänzungslichts als auch basierend auf dem vom Erkennungselement erkannten Instrumententyp erfolgt.

Beispielsweise kann der Speicher sogenannte Look-Up-Tables enthalten, auf denen Sollwerte je Instrumententyp abgespeichert sind, z.B. 23G IPDmax = 0,7 mA. Auf dem erkennbaren Element wie dem RFID-Tag des Lichtinstruments wird der Instrumententyp gespeichert. Dieser Speicherwert wird durch eine Antenne der ophthalmischen Beleuchtungsvorrichtung erkannt und ausgelesen. Die Information verwendet der Speicher, um den Referenzwert je Instrumententyp zu berechnen. Da unterschiedliche Gauge-Grössen der Lichtinstrumente unterschiedliche Einkopplungseffizienzen aufweisen, umfasst die Look-Up-Table vorzugsweise empirisch ermittelte Effizienzwerten je nach Gauge-Grösse. Beispielseise seien maximal 20 lm gewünscht, was hier 100% entspricht. Um diese 20 lm zu erreichen, muss abhängig vom Instrumententyp unterschiedlich viel optische Leistung am Ort der Fasereinkopplung bereitgestellt werden, z.B. um mit einer Faser von d = 200 $\mu$m 20 lm zu erreichen wird deutlich mehr optische Leistung benötigt als bei einer Faser von d = 500 $\mu$m. Denn je grösser die Faser, desto mehr Licht kann eingekoppelt werden, was der Kopplungseffizienz entspricht. Als weiteres Beispiel sei hier die Erkennung eines ophthalmischen Lichtinstruments mit einer Faser von 300 $\mu$m Durchmesser genannt, wobei aufgrund dessen festgelegt ist, dass bei diesem Instrumententyp ein maximales Referenzsignal von IPDmax = 1,2 mA nicht überschritten werden soll, was den maximalen 20 lm entspricht. Wünscht der Benutzer nun einen Lichtstrom von 10 lm, kann er eine Einstellung auf 50 % wählen, so dass die Leistungsregelung die Leistung der Leuchtquellen mit dem neuen Soll-Wert von $I_{PD-SOLL}$ = 1,2 mA * 0,5 = 0,6 mA regelt.

Dadurch gestattet die ophthalmische Beleuchtungsvorrichtung eine Leistungsregelung in Abhängigkeit vom emittierten Lichtstrom der jeweiligen Leuchtquelle und der Instrumentengrösse des ophthalmischen Lichtinstruments. Dies gestattet eine Abgabe der phototoxischen Dosis ins Auge und gewährleistet, dass nicht zuviel Lumen beziehungsweise eine zu hohe Lichtleistung ins ophthalmische Lichtinstrument und folglich ins Auge abgegeben wird.

In einem zweiten Aspekt wird eine ophthalmische Beleuchtungsvorrichtung zur Beleuchtung eines ophthalmischen Lichtinstruments angegeben, welche mindestens eine Leuchtquelle zur Erzeugung von Beleuchtungslicht, und mindestens ein Sensorelement zur Erfassung einer Strahlungsleistung des Beleuchtungslichts umfasst. Das Sensorelement ist teilweise in einem Strahlengang des Beleuchtungslichts angeordnet. Die ophthalmische Beleuchtungsvorrichtung ist dazu ausgebildet, ein auf der erfassten Strahlungsleistung basiertes Regelsignal an die Leuchtquelle zur Leistungsregelung der Leuchtquelle zu senden.

Sämtliche Aussagen betreffend die ophthalmische Beleuchtungsvorrichtung gemäss dem ersten Aspekt treffen vorzugsweise analog auf die ophthalmische Beleuchtungsvorrichtung gemäss dem zweiten Aspekt zu und umgekehrt.

Das heisst, bei der Leuchtquelle handelt es sich vorzugsweise um die obige Anregungsquelle und/oder eine oder mehrere der ergänzenden Leuchtquellen. Weiter kann die ophthalmische Beleuchtungsvorrichtung auch mindestens ein Leuchtelement aufweisen.

Genauso denkbar ist jedoch, dass kein photolumineszierendes Leuchtelement vorhanden ist und stattdessen Beleuchtungslicht einzig basierend auf der mindestens einer Leuchtquelle erzeugt wird.

In einem dritten Aspekt wird eine ophthalmische Beleuchtungsvorrichtung zur Beleuchtung eines ophthalmischen Lichtinstruments angegeben, welche mindestens eine Leuchtquelle zur Erzeugung von Beleuchtungslicht umfasst. Die ophthalmische Beleuchtungsvorrichtung umfasst weiter mindestens ein Erkennungselement zum Erkennen eines erkennbaren Elements eines mit der ophthalmischen Beleuchtungsvorrichtung verbundenen ophthalmischen Lichtinstruments. Die ophthalmische Beleuchtungsvorrichtung ist dazu ausgebildet, ein auf dem erkannten erkennbaren Element basiertes Regelsignal an die Leuchtquelle zur Leistungsregelung der Leuchtquelle zu senden. Das erkennbare Element basiert vorzugsweise auf einem Instrumententyp des ophthalmischen Lichtinstruments.

Sämtliche Aussagen betreffend die ophthalmische Beleuchtungsvorrichtung gemäss dem dritten Aspekt treffen vorzugsweise analog auf die ophthalmische Beleuchtungsvorrichtung gemäss dem ersten Aspekt und dem zweiten Aspekt zu und umgekehrt.

In einem weiteren Aspekt wird ein ophthalmisches Beleuchtungssystem umfassend eine ophthalmische Beleuchtungsvorrichtung wie oben beschrieben und ein ophthalmisches Lichtinstrument angegeben, wobei das ophthalmische Lichtinstrument zum Zuleiten von Beleuchtungslicht in den intraokularen Raum eines Auges ausgebildet ist.

Sämtliche vorherige Aussagen betreffend das ophthalmische Lichtinstrument treffen vorzugsweise auf das hierin genannte ophthalmische Lichtinstrument zu.

Vorzugsweise handelt es sich beim ophthalmische Lichtinstrument um ein allgemein bekanntes ophthalmische Lichtinstrument, welches einen allgemein bekannten Lichtinstrument-Typen aufweist, wie beispielsweise ein 20G, 23G, 25G, und 27G Typ, wobei "G" für Gauge steht. Vorzugsweise umfasst das ophthalmische Lichtinstrument einen Lichtleiter, beispielsweise in Form einer Faser, wobei das Beleuchtungslicht der Beleuchtungsvorrichtung in den Lichtleiter gekoppelt wird und dadurch zu einer Beleuchtung des ophthalmischen Lichtinstruments führt.

Weiter ist es bevorzugt, dass das ophthalmische Lichtinstrument mindestens ein erkennbares Element aufweist, welches vom Erkennungselement der ophthalmischen Beleuchtungsvorrichtung wie oben beschrieben erkannt werden kann. Vorzugsweise umfasst das ophthalmische Lichtinstrument einen Stecker zur Verbindung mit einer Buchse der ophthalmischen Beleuchtungsvorrichtung. Insbesondere bevorzugt ist das erkennbare Element des ophthalmischen Lichtinstruments am Stecker angeordnet.

KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:

Fig. 1　　zeigt eine perspektivische Ansicht eines ophthalmischen Beleuchtungssystems umfassend eine ophthalmische Beleuchtungsvorrichtung und ein ophthalmisches Lichtinstrument;

Fig. 2　　zeigt eine perspektivische Ansicht einer Buchse der ophthalmischen Beleuchtungsvorrichtung gemäss Figur 1, wobei ein Erkennungselement an der Buchse angeordnet ist;

Fig. 3　　zeigt eine teilweise Explosionsansicht der Buchse gemäss Figur 2;

Fig. 4　　zeigt eine perspektivische Ansicht des ophthalmisches Lichtinstruments gemäss Figur 1;

Fig. 5　　zeigt eine teilweise Explosionsansicht der ophthalmischen Beleuchtungsvorrichtung gemäss Figur 1;

Fig. 6　　zeigt eine Schnittansicht durch die ophthalmische Beleuchtungsvorrichtung gemäss Figur 5;

Fig. 7　　zeigt eine schematische Erzeugung von Beleuchtungslicht mittels einer ophthalmischen Beleuchtungsvorrichtung gemäss einer ersten Ausbildung;

Fig. 8　　zeigt eine schematische Erzeugung von Beleuchtungslicht mittels einer ophthalmischen Beleuchtungsvorrichtung gemäss einer weiteren Ausbildung;

Fig. 9　　zeigt eine schematische Erzeugung von Beleuchtungslicht mittels einer ophthalmischen Beleuchtungsvorrichtung gemäss einer weiteren Ausbildung;

Fig. 10　　zeigt ein normalisiertes photometrisches Spektrum von Beleuchtungslicht der ophthalmischen Beleuchtungsvorrichtung;

Fig. 11　　zeigt eine schematische Erzeugung von Beleuchtungslicht mittels einer ophthalmischen Beleuchtungsvorrichtung gemäss einer weiteren Ausbildung;

Fig. 12　　zeigt ein Diagramm des mittels der ophthalmischen Beleuchtungsvorrichtung erzeugten Lichtstroms in Abhängigkeit von deren Abbildungsmassstab;

Fig. 13　　zeigt eine schematische Erzeugung von Beleuchtungslicht mittels einer ophthalmischen Beleuchtungsvorrichtung gemäss einer weiteren Ausbildung;

Fig. 14　　zeigt eine schematische Erzeugung von Beleuchtungslicht mittels einer ophthalmischen Beleuchtungsvorrichtung gemäss einer weiteren Ausbildung;

Fig. 15　　zeigt ein Diagramm des mittels einer ergänzenden Leuchtquelle der ophthalmischen Beleuchtungsvorrichtung erzeugten Lichtstroms in Abhängigkeit von deren Abbildungsmassstab für ophthalmische Lichtinstrumente verschiedener Fasergrössen;

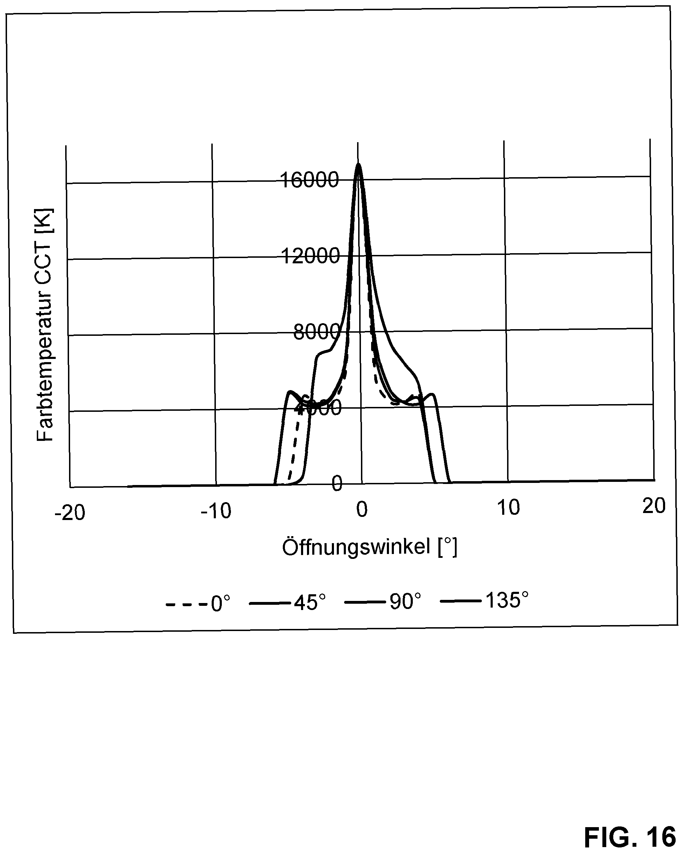

Fig. 16　　zeigt ein Diagramm der Farbtemperatur des mittels der ophthalmischen Beleuchtungsvorrichtung erzeugten Beleuchtungslichts über dessen Öffnungswinkel;

Fig. 17 zeigt ein weiteres Diagramm der Farbtemperatur des mittels der ophthalmischen Beleuchtungsvorrichtung erzeugten Beleuchtungslichts über dessen Öffnungswinkel, gemessen mit einem 25G Panorama Lichtinstruments;

Fig. 18 zeigt ein Diagramm der Transmission bzw. Reflexion in Abhängigkeit einer Wellenlänge eines optischen Filterelements der ophthalmischen Beleuchtungsvorrichtung;

Fig. 19 zeigt ein Diagramm der Gefährdungskurve nach EN ISO 15004-2:2007;

Fig. 20 zeigt einen schematischen Regelkreis des ophthalmischen Beleuchtungssystems.

## BESCHREIBUNG BEVORZUGTER AUSFÜHRUNGSFORMEN

Aspekte der erfindungsgemässen ophthalmischen Beleuchtungsvorrichtung 1 bzw. des erfindungsgemässen Beleuchtungssystems 100 umfassend die ophthalmische Beleuchtungsvorrichtung 1 sowie ein ophthalmisches Lichtinstrument 2 werden nun anhand der Figuren illustriert.

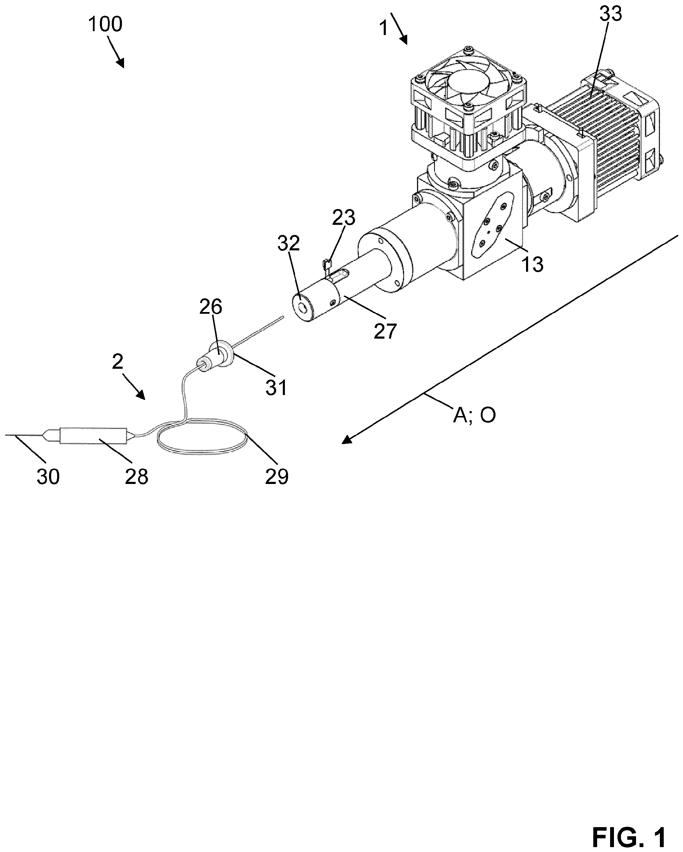

Wie aus Figur 1 hervorgeht, weist das ophthalmische Beleuchtungssystem 100 also eine ophthalmische Beleuchtungsvorrichtung 1 sowie ein damit verbindbares ophthalmisches Lichtinstrument 2 auf, wobei das ophthalmische Lichtinstrument 2 zum Zuleiten von Beleuchtungslicht 6 der ophthalmischen Beleuchtungsvorrichtung an eine chirurgische Stelle im Auge ausgebildet ist.

Das Lichtinstrument 2 umfasst einen Stecker 26 und die Beleuchtungsvorrichtung 1 umfasst eine Buchse 27. Die Beleuchtungsvorrichtung 1 umfasst ein Gehäuse 13 und die Buchse 27 ist am Gehäuse 13 fest befestigt. Das Lichtinstrument 2 ist hier beabstandet von der Buchse 27 der Beleuchtungsvorrichtung 1 dargestellt. Das Lichtinstrument 2 kann über dessen Stecker 26 mit der Buchse 27 der Beleuchtungsvorrichtung 1 verbunden werden. Das Lichtinstrument 2 umfasst in der gezeigten Ausführungsform weiter einen Handgriff 28, und einen Lichtleiter 29 in Form einer Faser. Der Lichtleiter 29 steht mit dem Stecker 26 des Lichtinstruments 2 in fester Verbindung und Beleuchtungslicht 6 von der Beleuchtungsvorrichtung 1 wird in den Lichtleiter 29 eingekoppelt. Der Handgriff 28 umfasst zudem eine Kanüle 30, welche in das Patientenauge geführt werden kann. Die Lichtfaser ist mit der Kanüle verbunden und koppelt das Beleuchtungslicht 6 am Ende der Kanüle aus dem Lichtinstrument 2 aus. Im hier gezeigten Beispiel ist der Stecker 26 des Lichtinstruments 2 magnetisch anziehbar bzw. anziehend ausgebildet und weist eine Anschlagsfläche 31 auf und die Buchse 27 der Beleuchtungsvorrichtung 1 ist ebenfalls magnetisch anziehbar bzw. anziehend ausgebildet und weist eine Anschlagsfläche 32 auf. Aufgrund einer magnetischen Anziehungskraft werden der Stecker 26 und die Buchse 27 über ihre jeweiligen Anschlagsflächen 31; 32 miteinander lösbar verbunden.

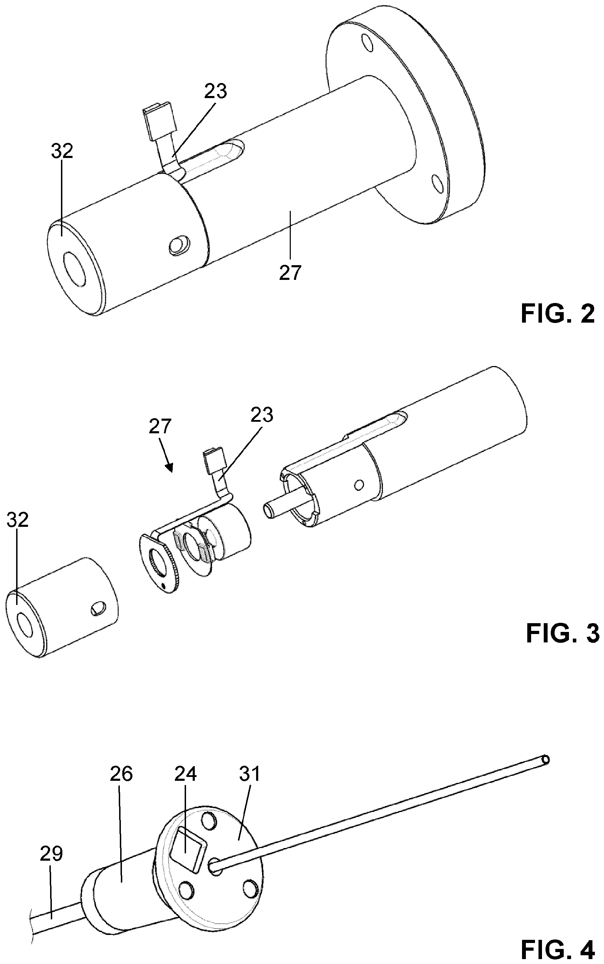

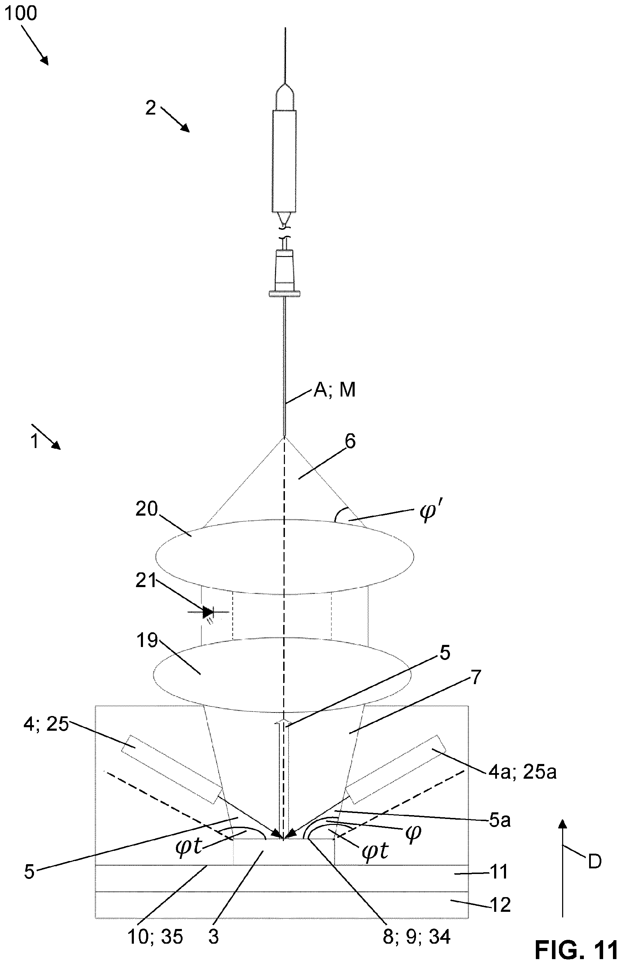

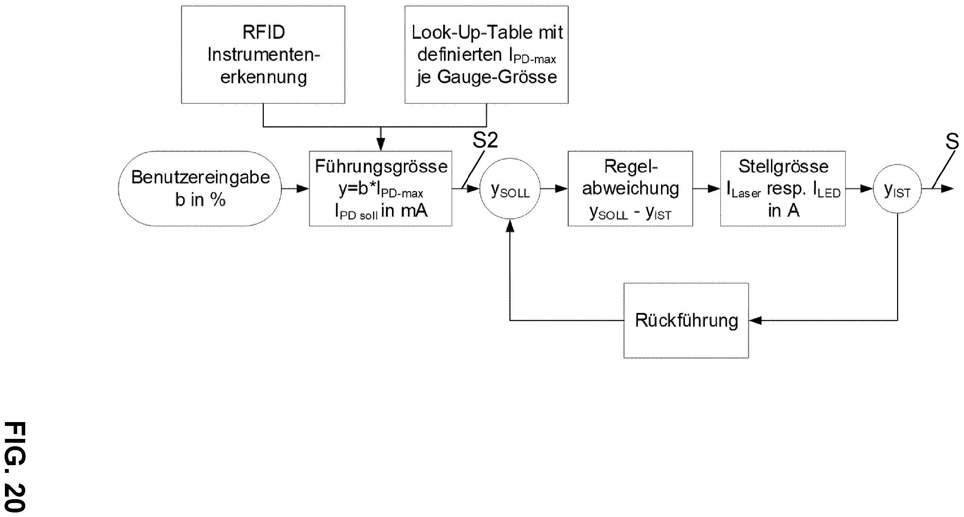

Wie aus den Figuren 2 bis 4 hervorgeht, weist die Beleuchtungsvorrichtung 1 ein Erkennungselement 23 zum Erkennen eines erkennbaren Elements 24 des Lichtinstruments 2 auf. Das Erkennungselement 23 ist hier an der Buchse 27 der Beleuchtungsvorrichtung 1 und das erkennbare Element 24 des Lichtinstruments 2 ist an dessen Stecker 26 angeordnet. In diesem Beispiel ist das Erkennungselement 23 eine RFID-Antenne und das erkennbare Element 24 ein RFID-Tag, wobei der RFID-Tag 24 Informationen zum Instrumententyp des Lichtinstruments 2 aufweist, und der Beleuchtungsvorrichtung 1 gestattet, eine Regelung basierend auf dem erkannten Instrumententyp durchzuführen, welche im Zusammenhang mit Figur 20 eingehend erläutert wird. Wie insbesondere aus den Figuren 11, 13 und 14 hervorgeht, umfasst die Beleuchtungsvorrichtung 1 auch mindestens ein Sensorelement 21 zur Erfassung einer Strahlungsleistung der Beleuchtungsvorrichtung 1 und ermöglicht eine Leistungsregelung basierend auf der erfassten Strahlungsleistung, siehe Erläuterungen zur Regelung im Zusammenhang mit Figur 20. Das heisst, und wie aus diesen Figuren hervorgeht umfasst die Beleuchtungsvorrichtung 1 Sensorelemente 21, welche teilweise im Strahlengang der Leuchtquellen, hier im Strahlengang des Beleuchtungslichts 6 und, falls vorhanden optional auch im Strahlengang von Anregungslicht 5 bzw. von Ergänzungslichts 18 (siehe weiter unten) angeordnet ist. Insbesondere sind die Sensorelemente 21 im Randbereich der jeweiligen Strahlengänge angeordnet und dazu ausgebildet, eine Strahlungsleistung des jeweiligen Strahlengangs zu erfassen. Weiter ist die Beleuchtungsvorrichtung 1 dazu ausgebildet, ein auf der erfassten Strahlungsleistung basiertes Regelsignal S an die Anregungsquelle 4 sowie, falls vorhanden an ergänzende Leuchtquellen 17 zur Leistungsregelung der Anregungsquelle 4 und gegebenenfalls der ergänzenden Leuchtquellen 17 zu senden.

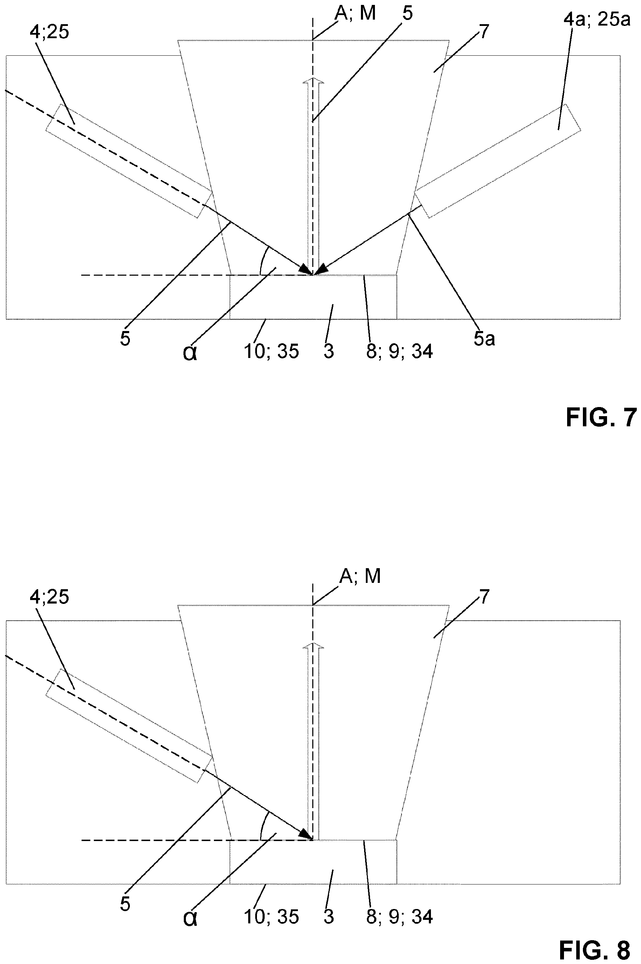

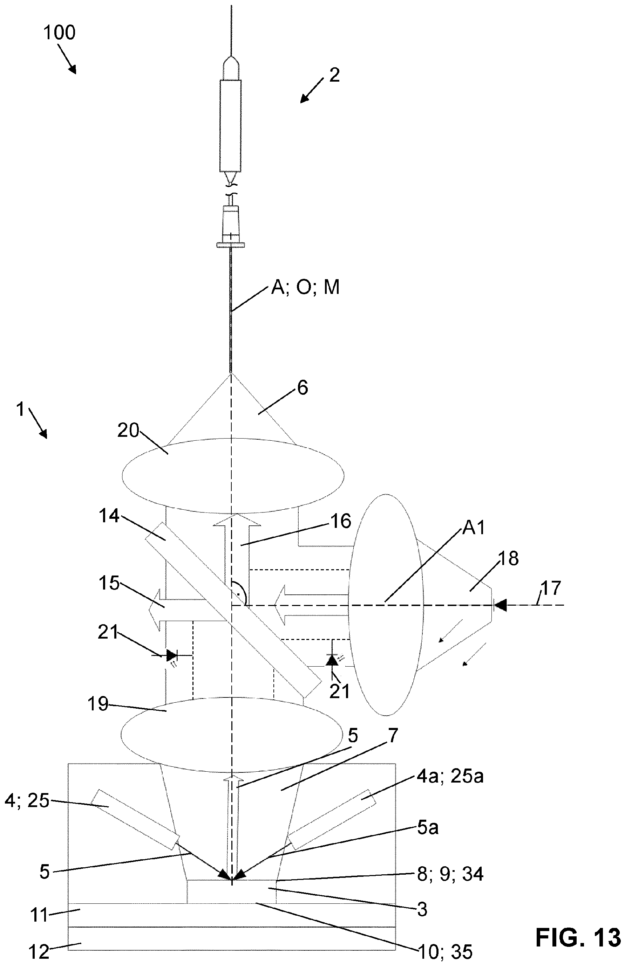

Wie aus den Figuren 1, 5 und 6 hervorgeht, weist die Beleuchtungsvorrichtung 1 ein Gehäuse 13 auf, in welchem eine oder mehrere Leuchtquellen 4; 4a; 17; 17a; 25; 25a sowie weiter ein Leuchtelement 3, ein Wärmeleitelement 11 und ein Kühlelement 12 angeordnet sind, siehe auch Figuren 7-9, 11, und 13-14. Wie nachfolgend noch eingehend diskutiert wird, kann es sich bei der Leuchtquelle 25 um eine Anregungsquelle 4 zur Anregung des Leuchtelements 3 mittels Anregungslicht 5 aus stimulierter Emission handeln und das Leuchtelement 3 kann photolumineszierend sein. In den hier gezeigten Beispielen ist das Leuchtelement 3 schichtförmig und besteht aus mindestens einem photolumineszierenden Material. In diesem Fall erzeugt die Beleuchtungsvorrichtung 1 Beleuchtungslicht 6 umfassend oder bestehend aus der Photolumineszenz 7 des Leuchtelements 3. Weitere Leuchtquellen 25 wie ergänzende Leuchtquellen 17; 17a können ebenfalls vorhanden sein und werden im Zusammenhang mit den Figuren 13-14 eingehender erläutert. Das Wärmeleitelement 11 leitet Wärme des Anregungslichts 5, welche beim Anregen des Leuchtelements 3 erzeugt wird, zum Kühlelement 12, wobei das Kühlelement 12 die Wärme an die Umgebung abgibt. Im vorliegenden Fall ist das Wärmeleitelement 11 eine Kupfer- oder Aluminiumschicht. Auch das Kühlelement 12 umfasst eine Kupfer- oder Aluminiumschicht, welche hier zudem mit einem Lüfter 33 in Verbindung steht. Wie aus Figur 1 hervorgeht, ist der Lüfter 33 aussenseitig am Gehäuse 13 der Beleuchtungsvorrichtung 1 angeordnet und fest mit diesem verschraubt.

Nachfolgend werden nun verschiedene Ausbildungen der Beleuchtungsvorrichtung 1, insbesondere der Leuchtquellen 4; 4a; 17; 17a; 25; 25a und das damit erzeugte Beleuchtungslicht 6, diskutiert.

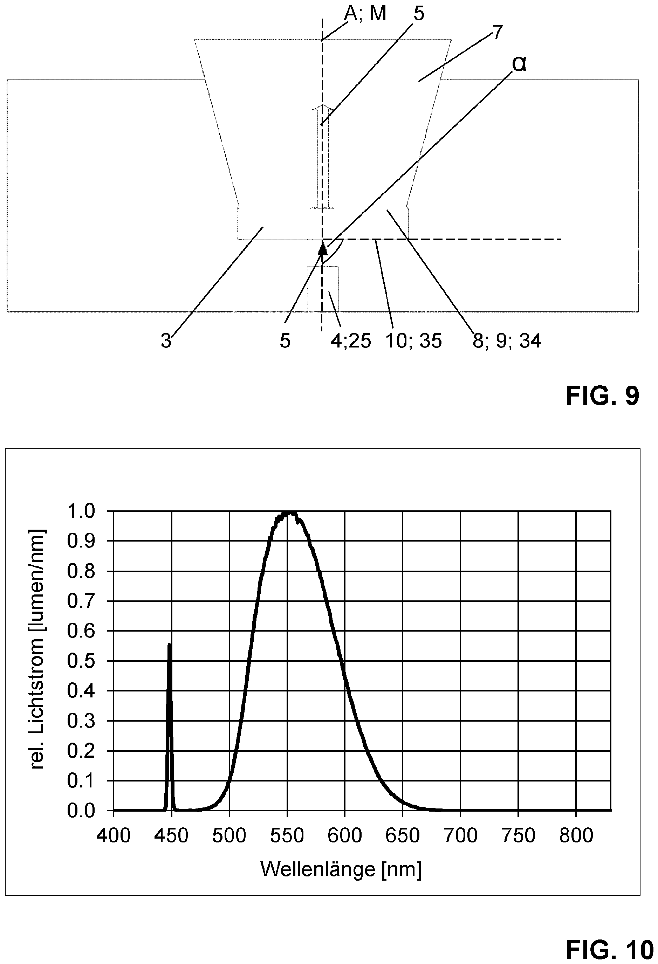

Wie aus den Figuren 7-9, 11 und 13-14 hervorgeht, umfasst die Beleuchtungsvorrichtung 1 jeweils eine oder zwei Anregungsquellen 4; 4a, welche in einem Winkel $\alpha$ zum Leuchtelement 3, insbesondere zu einer Fläche 8 des Leuchtelements 3, angeordnet sind. Diese Anregungsquellen 4; 4a emittieren dabei Anregungslicht 5; 5a, welches ebenfalls im Winkel $\alpha$ zum Leuchtelement 3, insbesondere zur Fläche 8 des Leuchtelements 3, angeordnet ist. Mit Ausnahme von Figur 9 sind die Anregungsquellen 4; 4a bzw. deren Anregungslicht 5; 5a dabei jeweils in einem Winkel $\alpha$ von 45° zum Leuchtelement 3 bzw. dessen Fläche 8 angeordnet. In der Beleuchtungsvorrichtung 1 gemäss Figur 9 hingegen ist die Anregungsquelle 4 und deren Anregungslicht 5 in einem Winkel $\alpha$ von 90° zum Leuchtelement 3 bzw. dessen Fläche 8 angeordnet. Im ersten Fall ist die Fläche 8 des Leuchtelements 3 jeweils eine Frontfläche 34 des Leuchtelements 3, welche hier eine Frontseite 9 des Leuchtelements 3 bereitstellt. Hier ist bzw. sind die Anregungsquellen 4; 4a bezüglich einer Ausbreitungsrichtung A der Photolumineszenz 7 gesehen nach dem Leuchtelement 3 angeordnet. Im zweiten Fall ist die Fläche 8 eine Rückfläche 35 des Leuchtelements 3, welche hier eine Rückseite 10 des Leuchtelements 3 bereitstellt. Hier ist also die Anregungsquelle 4 bezüglich der Ausbreitungsrichtung A gesehen vor dem Leuchtelement 3 angeordnet. Die Ausbreitungsrichtung A der Photolumineszenz 7 ist parallel zu einer Flächennormalen des Leuchtelements 3 und senkrecht auf die Fläche 8 des Leuchtelements 3. Wie in den Figuren 7, 10 und 13-14 ersichtlich ist, sind die beiden Anregungsquellen 4; 4a jeweils bezüglich der Ausbreitungsrichtung A gesehen einander gegenüberliegend angeordnet, und zwar bezüglich einer zentral durch das Leuchtelement verlaufenden Mittelachse M einander gegenüberliegend angeordnet.

Je nach Anordnung der Anregungsquelle 4; 4a in Bezug auf das Leuchtelement 3 wird zumindest ein Teil des Anregungslichts 5; 5a vom Leuchtelement 3 reflektiert (siehe Figuren 7-8) respektive durch das Leuchtelement 3 transmittiert (siehe Figur 9), wobei Beleuchtungslicht 6 umfassend ein Kombinationsspektrum aus dem Anregungslicht 5; 5a und der Photolumineszenz 7 gebildet wird.

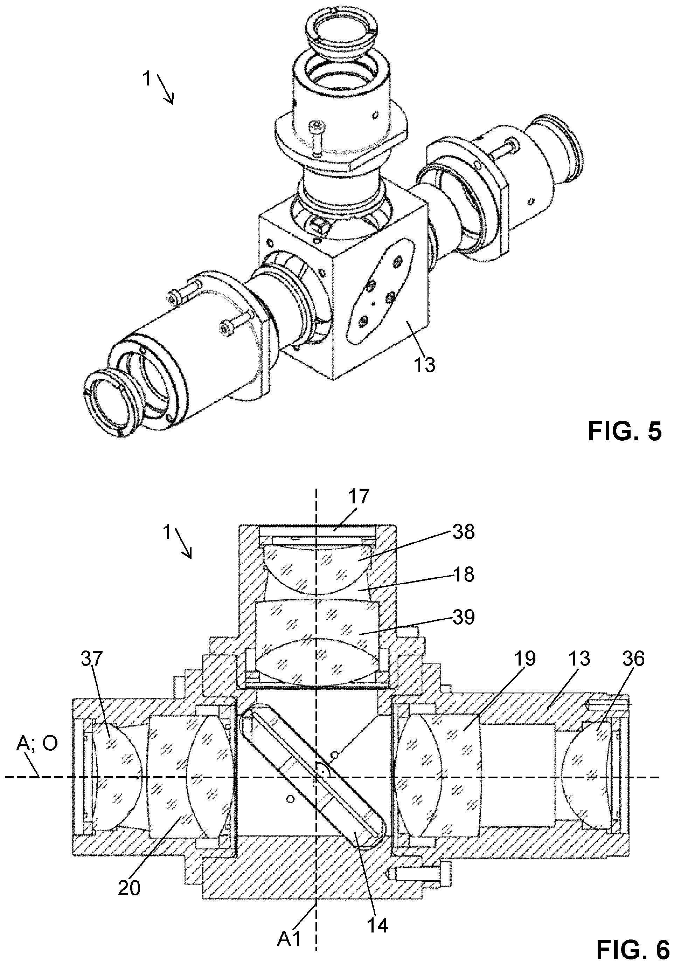

Wie in den Figuren 6 und 13-14 ersichtlich ist, kann die Beleuchtungsvorrichtung 1 mindestens ein optisches Filterelement 14 umfassen, welches einfallendes Licht wie zum Beispiel das reflektierte bzw. transmittierte Anregungslicht 5; 5a und/oder die Photolumineszenz 7 basierend auf einer optischen Eigenschaft des einfallenden Lichts als Reflexionslicht 15 reflektiert und als Transmissionslicht 16 transmittiert. Im vorliegenden Fall handelt es sich beim optischen Filterelement 14 um einen dichroitischen Spiegel. Abhängig von einer Wellenlänge des einfallenden Lichts 5; 5a; 7 und davon, ob das vom optischen Filterelement 14 reflektierte oder transmittierte Licht 15; 16 verwendet wird, kann Beleuchtungslicht 6 von unterschiedlicher Wellenlänge erzeugt werden. Verständnishalber werden hierin das alsdann verwendete Licht als Ausgangslicht und das nicht verwendete Licht als Ausschusslicht bezeichnet. Das optische Filterelement 14 ist entlang der Ausbreitungsrichtung A der Photolumineszenz 7 gesehen nach dem Leuchtelement 3 angeordnet sowie im Gehäuse 13 angeordnet, siehe Figur 6. Wie weiter aus Figur 6 hervorgeht, umfasst die Beleuchtungsvorrichtung 1 eine Kollektorlinse 19 und eine Kondensorlinse 20. Die Kollektorlinse 19 fängt die Photolumineszenz 7 des Leuchtelements 3 auf und strahlt sie entlang einer optischen Achse O entlang der Beleuchtungsvorrichtung 1 in Richtung der Kondensorlinse 20. Die Kondensorlinse 20 gibt das Beleuchtungslicht 6 aus der Beleuchtungsvorrichtung 1 und in das ophthalmische Lichtinstrument 2 aus. Im vorliegenden Fall bilden die Kollektorlinse 19 und die Kondensorlinse 20 jeweils ein achromatisches Linsenpaar. Wie weiter aus Figur 6 hervorgeht, umfasst die Beleuchtungsvorrichtung 1 weitere optische Elemente, beispielsweise eine Kollimationslinse 36 zur Kollimation der Photolumineszenz 7 und von reflektiertem Anregungslicht 5; 5a, sowie eine Fokuslinse 37 zur Fokussierung des erzeugten Beleuchtungslichts 6 auf das Lichtinstrument 2. Kollimationslinse 36 und Kollektorlinse 19 bilden zusammen die Kollektor-Optik-Gruppe. Kondensorlinse 20 und Fokuslinse 37 bilden zusammen die Kondensor-Optik-Gruppe. Wie nachfolgend noch erörtert wird sind auch Kollimationslinsen 38; 38a zur Kollimation von Ergänzungslicht 18; 18a von ergänzenden Leuchtquellen 17; 17a respektive achromatische Linsenpaare 39, 39a vorhanden, siehe Figuren 6, 13 und 14. Diese Optiken (38; 38a sowie 39; 39a) der ergänzenden Leuchtquellen bilden jeweils wiederum Kollektor-Optik-Gruppen. Die optische Achse O ist parallel zur Ausbreitungsrichtung A der Photolumineszenz 7, zur Flächennormalen auf die Frontfläche 34 des Leuchtelements 3 sowie zur Mittelachse M durch das Leuchtelement 3. Das optische Filterelement 14 ist auf der optischen Achse O angeordnet.

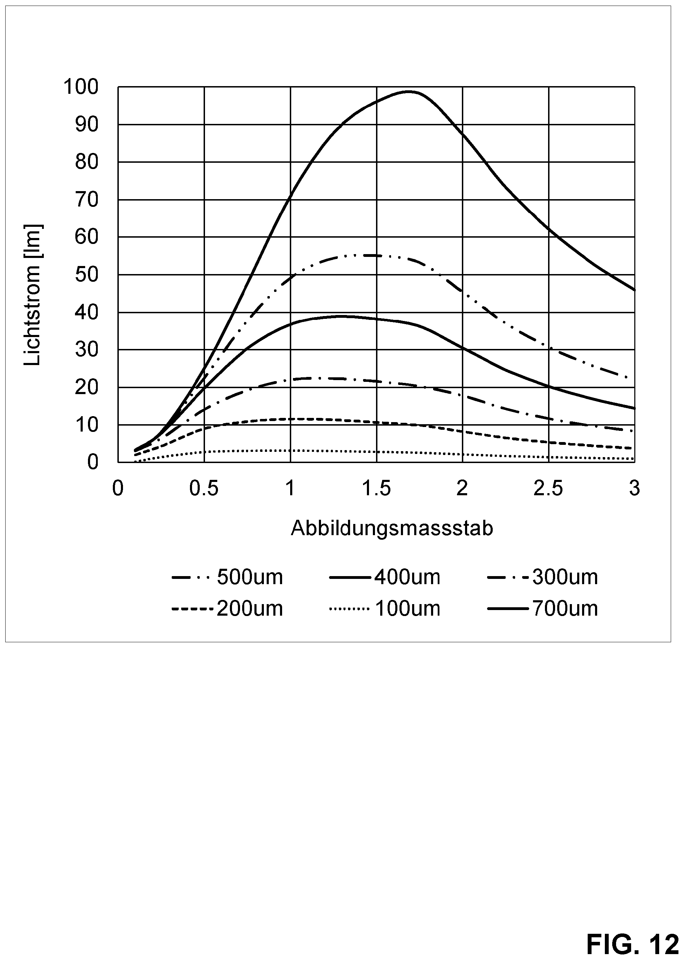

Wie aus Figur 11 hervorgeht, bildet das aus der ophthalmischen Beleuchtungsvorrichtung abgestrahlte Beleuchtungslicht objektseitige Abstrahlwinkel φ, φt und das durch die ophthalmische Beleuchtungsvorrichtung zu beleuchtende ophthalmische Lichtinstrument definiert einen bildseitigen Abstrahlwinkel φ'. Bei den objektseitigen Abstrahlwinkeln handelt es sich um die Lichtstärkeverteilungskurve der Leuchtquelle. Dabei wird zwischen einem tatsächlichen objektseitigen Abstrahlwinkel φt und dem genutzten objektseitigen Abstrahlwinkel φ unterschieden. Würde man den tatsächlichen objektseitigen Abstrahlwinkel φt nutzen, würde man das Lichtinstrument, insbesondere dessen Faser, überstrahlen, und dadurch viel Licht verlieren. Diese Beziehung ist in Figur 12 dargestellt. Wie aus dem Diagramm hervorgeht, verliert man entweder Licht, wenn nicht der tatsächliche objektseitige Abstrahlwinkel verwendet wird, oder man verliert Licht weil die Fasern überstrahlt werden. In der vorliegenden Erfindung sind diese Grössen derart, dass ein optimales Verhältnis gebildet wird. Im gezeigten Beispiel wird ein ophthalmisches Lichtinstrument umfassend eine Faser mit NA von 0.5 sowie eine Leuchtquelle mit einer Lichtstärkeverteilungskurve von etwa 120° FWHM verwendet.

Wie aus den Figuren 13-14 hervorgeht, umfasst auf das optische Filterelement 14 einfallende Licht die Photolumineszenz 7 sowie Anregungslicht 5; 5a, das vom Leuchtelement 3 reflektiert wird bzw. durch das Leuchtelement 3 transmittiert wird.

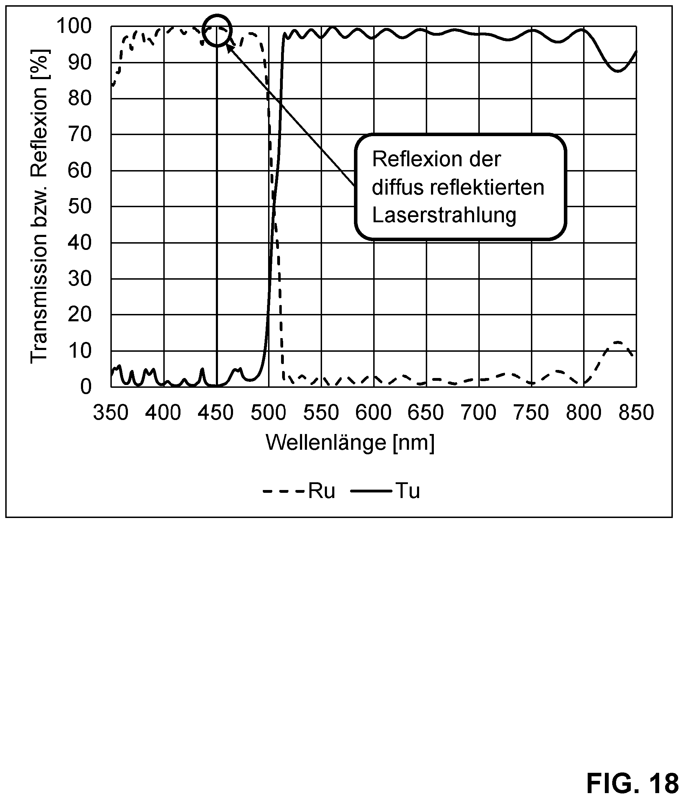

Das optische Filterelement 14 wie hier der dichroitische Spiegel dient zudem der Filterung von phototoxischem Licht, beispielsweise von Anregungslicht 5; 5a mit einer Wellenlänge in einem für das Auge schädlichen Wellenlängenbereich. Wie erwähnt kann eine Anregung des Leuchtelements 3 durch eine Anregungsquelle 4 in Form einer Laserdiode mit einer Wellenlänge des Anregungslichts 5; 5a von beispielsweise 450 Nanometer erfolgen. Diese Wellenlänge ist jedoch mit einer hohen phototoxischen Wirkung behaftet. Zur Reduzierung dieser Wirkung kann am Leuchtelement 3 diffus reflektiertes Laserlicht 5; 5a der Laserdiode durch den dichroitischen Spiegel 14 auf ein Absorptionselement wie beispielsweise eine Strahlfalle (nicht dargestellt) geleitet werden. Hierzu kann der dichroitische Spiegel 14 beispielsweise mit einer speziellen Schutzschicht versehen sein, die beim Peak der Laserstrahlung 5; 5a eine sehr hohe Reflexion von beispielsweise ≥99.5% aufweist, siehe kreisförmige Markierung in Figur 18 "Reflexion der diffus reflektierten Laserstrahlung". Das optische Filterelement 14 gewährt also eine Sicherheitsfunktion.

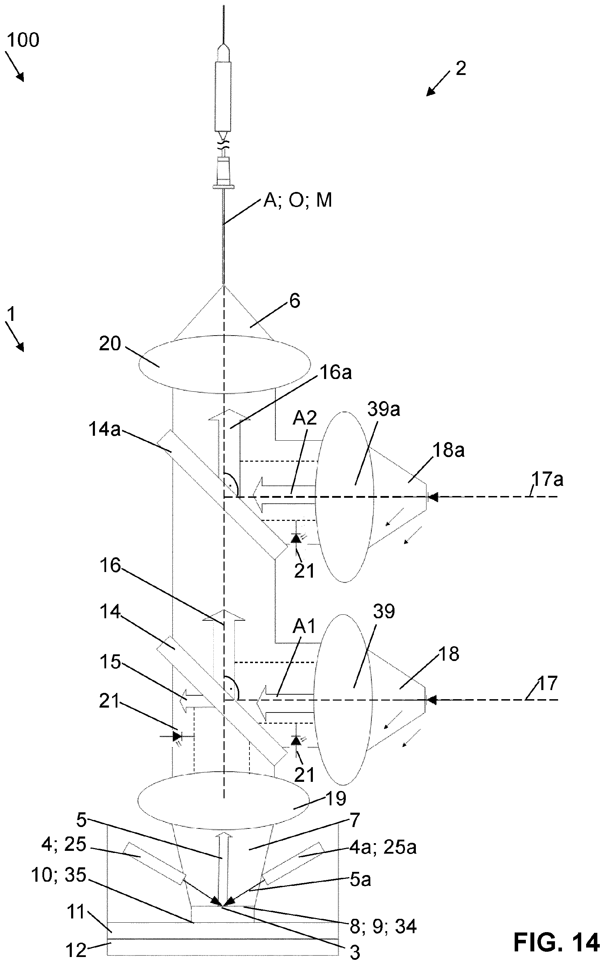

Wie aus den Figuren 13 und 14 hervorgeht und bereits erwähnt wurde, kann die Beleuchtungsvorrichtung 1 ergänzende Leuchtquellen 17, 17a zur Aussendung von Ergänzungslicht 18, 18a auf das optische Filterelement 14, 14a aufweisen derart, dass ein ergänztes Kombinationsspektrum umfassend hier das durch das optische Filterelement 14, 14a transmittierte Transmissionslicht 16 und das am optischen Filterelement 14, 14a reflektierte Ergänzungslicht 18, 18a gebildet wird. Wie aus diesen Figuren hervorgeht, sind die ergänzenden Leuchtquellen 18, 18a derart angeordnet, dass deren Ergänzungslicht 18, 18a jeweils entlang einer Ausbreitungsrichtung A1, A2 verläuft, welche senkrecht zur Ausbreitungsrichtung Ader Photolumineszenz 7 sowie zur optischen Achse O verläuft. Die zwei ergänzenden Leuchtquellen 17, 17a gemäss Figur 14 sind dabei parallel zueinander angeordnet und die Ausbreitungsrichtungen A1, A2 ihres jeweiligen Ergänzungslichts 18, 18a verlaufen ebenfalls parallel zueinander. Zudem weist diese Beleuchtungsvorrichtung 1 zwei optische Filterelemente 14, 14a auf, welche auf der optischen Achse O aufeinanderfolgend angeordnet sind.

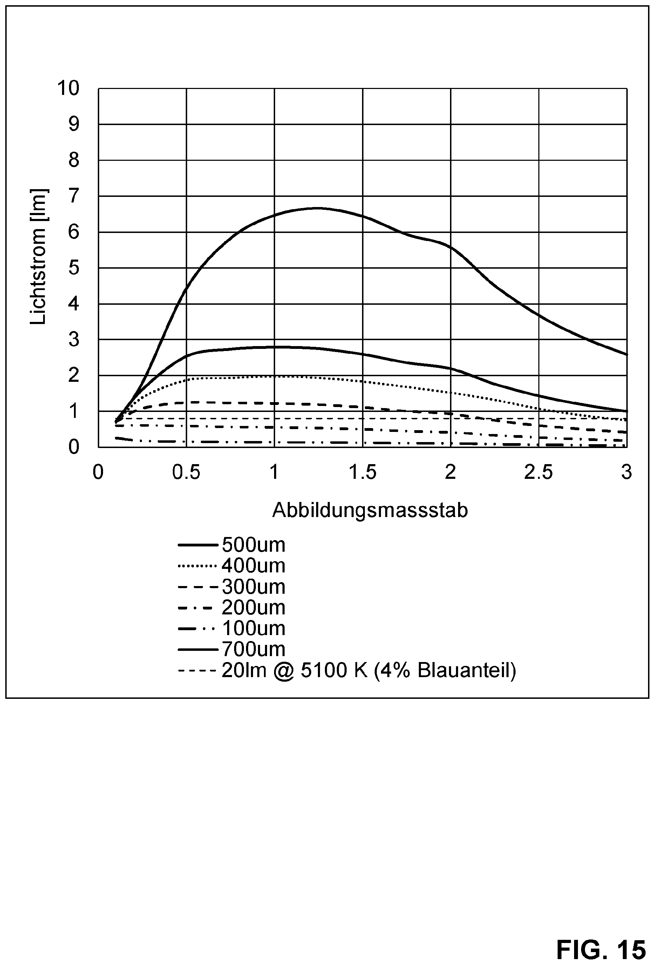

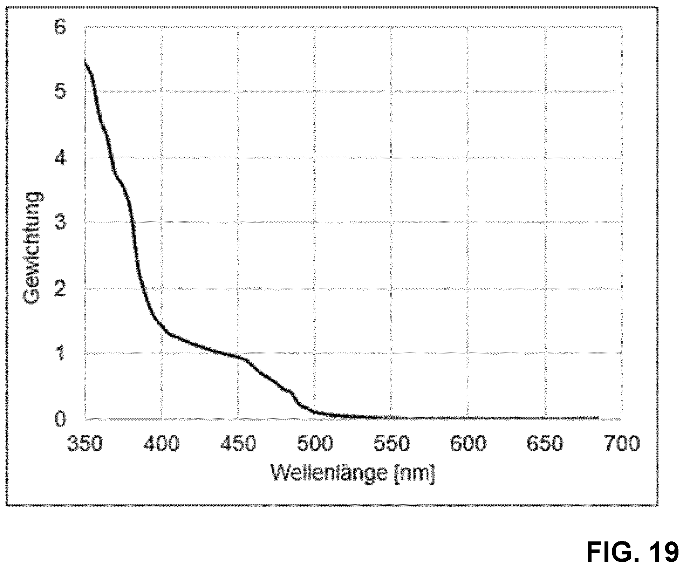

Da die Anregungsquelle 4 beispielsweise in Form der Laserdiode(n) und die ergänzenden Leuchtquellen 17, 17a beispielsweise in Form der LEDs unabhängig voneinander regelbar sind, siehe auch Erläuterungen weiter unten, kann der Anwender die Farbe des daraus kombinierten Beleuchtungslichts 6 von blau über weiss bis gelb einstellen. Dies ermöglicht die Farbeinstellung des Beleuchtungslichts 6 bei gleichzeitiger Minimierung der phototoxischen Wirkung. Figur 15 zeigt ein Diagramm des Lichtstroms einer ergänzenden Leuchtquelle, hier der ergänzenden LED, in Abhängigkeit von deren Abbildungsmassstab für Lichtinstrumente verschiedener Fasergrössen. Wie aus Figur 19 hervorgeht, führt ein Unterschied in der Wellenlänge vom Beleuchtungslicht 6 von beispielsweise 15 Nanometer im Falle einer Wellenlänge von 450 Nanometer gegenüber einer Wellenlänge von 465 Nanometer oder mehr zu einer Reduzierung der Gewichtung um mehr als 25 % gemäss der photochemischen Gefährdungsfunktion der EN ISO 15004-2:2007. Nämlich, wie dieser Graphik entnommen werden kann, hat eine Wellenlänge von 450 Nanometer eine Gewichtung der photochemischen Gefährdung von 0.94, während eine Wellenlänge von 465 Nanometer eine Gewichtung der photochemischen Gefährdung von 0.7 aufweist.

Nachdem nun hauptsächlich verschiedene strukturelle Aspekte einer erfindungsgemässen Beleuchtungsvorrichtung 1 diskutiert wurden, folgen nun Erklärungen zu möglichen Funktionsweisen und damit erreichten Eigenschaften.

So löst eine erfindungsgemässe Beleuchtungsvorrichtung 1 das Problem eines zu niedrigen Lichtstroms bei Lichtinstrumenten 2 mit kleinen Fasergrössen, beispielsweise von kleiner oder gleich 300 μm. Beispielsweise gestattet die Beleuchtungsvorrichtung 1 in Verbindung mit einem Leuchtinstrument 2 in Form eines 29G Endo-Illuminators einen Lichtstrom von 20 lm bei einer Farbtemperatur von 4000-6000 K. Derart hohe Lichtströme sind mit herkömmlichen LEDs nicht mehr erreichbar. In einer erfindungsgemässen Beleuchtungsvorrichtung 1 wird daher ein photolumineszierendes Leuchtelement 3 verwendet, welches nach Anregung mittels stimulierter Emission photoluminesziert. Der besondere Vorteil der Verwendung dieses Prinzips ist die höhere Leuchtdichte $[lm/(sr*m^2)]$. Dies bedeutet das mehr Licht pro Emitterfläche erzeugt wird. Dadurch lässt sich mehr Beleuchtungslicht 6 in kleine Fasern einkoppeln.

Dieses Prinzip wird in den Figuren 7 bis 10 illustriert, wobei jeweils eine Anregungsquelle 4, 4a in Form einer blau emittierenden Laserdiode ($\lambda$ = 450 Nanometer) zur Anregung eines Leuchtelements 3 verwendet wird, welches daraufhin gelb-grün photolumineszert. Da je nach Anordnung der Anregungsquelle 4, 4a in Bezug auf das Leuchtelement 3 das Anregungslicht 5, 5a vom Leuchtelement 3 reflektiert bzw. durch das Leuchtelement 3 transmittiert wird, entsteht Beleuchtungslicht 6 umfassend ein Kombinationsspektrum aus den blauen und gelbgrünen Spektren, was Weisslicht entspricht. Das entsprechende normalisierte photometrische Spektrum ist in Figur 10 gezeigt. Leistungsstarke LEDs können zwar einen ähnlich hohen oder gar höheren Lichtstrom erzeugen, weisen jedoch eine deutlich geringere Leuchtdichte auf. Dadurch ist der einkoppelbare Lichtstrom in v.a. kleinere Fasern stark begrenzt. Das Diagramm gemäss Figur 12 illustriert den Abbildungsmassstab des Leuchtelements 3 und damit die optimierte Einkopplungseffizienz der Beleuchtungsvorrichtung 1. Das heisst, das Leuchtelement 3 wird derart in die Beleuchtungsvorrichtung 1 integriert, dass eine Emitterfläche des Leuchtelements 3 - im speziellen die Anregungsfläche des Lasers auf der Leuchtstoffschicht - auf eine Faserendfläche fokussiert wird. Der gewählte Abbildungsmassstab weist dabei das optimale Verhältnis aus Strahlwinkel des Beleuchtungslichts 6, der Lichtstärkeverteilungskurve der Lumineszenz und der Bildgrösse auf. Ermittelt wurde dieser Wert durch Simulationen, welche durch Messungen an Prototypen verifiziert wurde. Figur 12 stellt die erreichbaren Lichtstromwerte am distalen Faserende, also dem der Beleuchtungsvorrichtung 1 abgewandten Ende der Faser 29, dar. Dabei wird der Abbildungsmassstab gegenüber dem erreichbaren Lichtstrom je Fasergrösse dargestellt.

Entscheidend ist die Etendue der Beleuchtungsvorrichtung 1, auch Lichtleitwert oder optischer Fluss genannt. Dieser spiegelt den Einfluss der geometrischen Grössen der Beleuchtungsvorrichtung 1 wider. Er ist eine Systemkonstante und berechnet sich aus dem Produkt von Raumwinkel und des Querschnitts der Faser. Der Lichtleitwert ist entscheidend für die einkoppelbare Lichtleistung bei fasergekoppelten optischen Systemen wie des vorliegenden Beleuchtungssystems 1. Wie aus Figur 12 hervorgeht, ergibt sich eine optimale Etendue respektive eine optimale Effizienz, vor allem bei kleinen Lichtfasern 29, bei einem Abbildungsmassstab von 1.25:1. Dementsprechend wird die Emitterfläche des Leuchtelements 3 von 1x1 mm vergrössert und die Lichtfasern 29 überstrahlt. Trotz der Überstrahlung ergibt sich im Falle einer Faser 29 mit NA von 0.5 eine optimale Lichteinkopplung. Die Lichtstromverluste bei 700 $\mu$m Fasern 29 sind nicht von Belang, da bei Vitrektomien von nicht mehr als 20 lm ausgegangen werden kann. Der Lichtstrom liegt hier mit ca. 88 lm ohnehin um ein Vielfaches über der Belastungsgrenze der Lichtfasern 29.

Die Photolumineszenz 7 des Leuchtelements 3 selbst erzeugt eine inhomogene Ausleuchtung hinsichtlich des Farbverteilung, wobei sich allfälliges Streulicht der Anregungsquelle 4, 4a in der Mitte des Lichtkegels mit einem engen Öffnungswinkel befinden würde, während die Photolumineszenz 7 einen grösseren Öffnungswinkel aufweist. Daher ist die Farbtemperatur in der Mitte des Lichtkegels des Beleuchtungslichts 6 deutlich höher als zum Rand hin. Beispielsweise hat sich ein Farbtemperaturunterschied von 2000-2500K von Zentrum zum Rand hin gezeigt. Zu Verringerung dieses Farbfehlers sieht die erfindungsgemässe Beleuchtungsvorrichtung 1 verschiedene Lösungen vor:

So wird eine farbhomogene Ausleuchtung mit linearer Farbregelung erhalten durch ein oder mehrere ergänzende Leuchtquellen 17, 17a, wie sie in den Figuren 13 und 14 gezeigt wird. So kann die Beleuchtungsvorrichtung 1 gemäss Figur 13 beispielsweise zwei Anregungsquellen 4, 4a in Form von blauen Laserdioden aufweisen, welche weiter eine ergänzende Leuchtquelle 17 in Form einer blauen LED zur Farbeinstellung aufweist. Je nach Einstellwert und Bestromung der Laserdioden 4, 4a und LED 17 ergibt sich an der Fasereinkopplung und somit letztlich im Auge blaues, weisses oder gelbes Beleuchtungslicht 6. Ebenso möglich ist eine Kombination mit einer grünen oder roten LED anstelle der blauen LED. Gemäss Figur 14 kann die Beleuchtungsvorrichtung 1 auch eine Kombination mit mehreren ergänzenden Leuchtquellen 17, 17a umfassen, beispielsweise eine blaue LED 17 sowie eine rote LED 17a. Dies erweitert den einstellbaren Farbraum für den Benutzer.

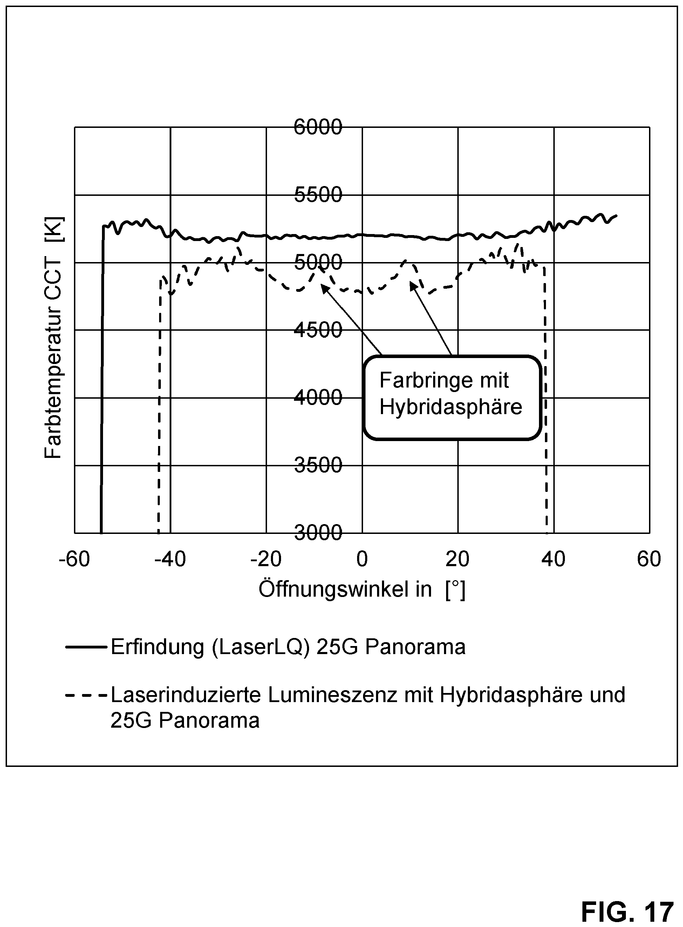

Die ophthalmische Beleuchtungsvorrichtung 1 erreicht eine sehr farbhomogene Beleuchtung mit Lichtinstrumenten der Vitrektomie. Dies ist auf die achromatischen Linsenpaare wie die Kollektorlinse 19 und die Kondensorlinse 20 zurückzuführen. Werden andere optische Elemente gewählt wie beispielsweise handelsübliche Hybridasphären zeigt sich die chromatische Aberration in Form von Farbringen. In Figur 17 wird die Beleuchtung mit einem 25G-Lichtinstrument 2 in Form von Farbtemperatur über dem Öffnungswinkel dargestellt. Wie aus dieser Figur hervorgeht, ist die Farbtemperatur der erfindungsgemässen Beleuchtungsvorrichtung 1 mit den achromatischen Linsenpaaren 19, 20 sehr konstant über einen Öffnungswinkel von $\geq 80°$ (siehe durchgezogene Linie), während die Verwendung von Hybridasphären anstelle der Achromate 19, 20 zwei Farbringe bei +/- 10° und +/- 30° aufweist (siehe gestrichelte Linie). Chirurginnen und Chirurgen empfinden solche Farbunterschiede als störend. Das heisst, mit einem gewöhnlichen Asphährensystem lässt sich der Farbtemperaturunterschied nicht homogenisieren, da Asphären lediglich den Fehler durch sphärische Aberration korrigieren. Die Erfindung hingegen löst diese Problematik durch das optische System mit achromatischen Linsenpaaren 19, 20, und gegebenenfalls weiter den ergänzenden Leuchtquellen 17, 17a und den optischen Filterelementen 14, 14a in Form des dichroitischen Spiegels. Diese konstante Farbtemperatur wird unabhängig von der Gauge-Grösse des Lichtinstruments 2 erhalten. Ohne geeignetes Linsensystem wie die hier verwendeten Achromaten 19, 20 weisen Lichtinstrumente 2 mit kleinem Faserdurchmesser zudem eine höhere Farbtemperatur auf als Lichtinstrumente 2 mit grösserem Faserdurchmesser. Das erfindungsgemässe optische System verhindert dies jedoch weitgehend. Durch die Kombination mit einer ergänzenden Leuchtquelle 17, 17a wie der weiteren blauen LED wie in Figur 13 dargestellt, lässt sich eine konstante Farbtemperatur bei einem Lichtstrom von 20 lm erreichen - unabhängig von der Fasergrösse des Lichtinstruments 2. Es liessen sich auch andere Farbtemperaturen wie bspw. 4000K oder 5000K bei identischem Lichtstrom mit unterschiedlichen Fasergrössen (20-29G) erzielen.

Wie eingangs erwähnt und in Figur 20 dargestellt, umfasst die ophthalmische Beleuchtungsvorrichtung 1 mindestens eine Regelungsschaltung. Die Regelungsschaltung ist mit dem oder den Sensorelementen 21 in Verbindung und dazu ausgebildet, die vom Sensorelement 21 erfasste Strahlungsleistung zu erhalten und darauf basierend ein erstes Regelsignal S zu ermitteln. Beispielsweise kann die Regelungsschaltung dazu ausgebildet sein, das erste Regelsignal S basierend auf einem Vergleich der erfassten Strahlungsleistung $y_{IST}$ mit Referenz-Strahlungsleistungen $y_{SOLL}$, also basierend auf einer Regelabweichung, zu ermitteln. Weiter ist die Regelungsschaltung mit der bzw. den Leuchtquellen 4; 4a; 17; 17a; 25; 25a in Verbindung und dazu ausgebildet, auf Basis des ermittelten ersten Regelsignal S die Stellgrösse der jeweiligen Leuchtquelle 4; 4a; 17; 17a; 25; 25a, hier als $I_{Laser}$ resp. $I_{LED}$ bezeichnet, in Form der Laserdiode 3, 3a resp. der ergänzenden Leuchtquelle in Form der LED 17, 17a zu übermitteln, welche daraufhin die Leistungen der von ihnen ausgesandten Lichts 5, 5a; 18, 18a entsprechend anpassen.. Wie ebenfalls auf Figur 20 hervorgeht, ist die Regelschaltung der Beleuchtungsvorrichtung 1 weiter dazu ausgebildet, ein auf dem erkannten erkennbaren Element 24 basiertes zweites Regelsignal S2 an die Anregungsquelle 4 und die ergänzende Leuchtquelle 17, 17a zur Leistungsregelung der Anregungsquelle 4 und der ergänzenden Leuchtquelle 17, 17a zu senden. Hier ist das erkennbare Element 24 ein Instrumententyp in Form einer Gauge-Grösse des ophthalmischen Lichtinstruments 2, welches über RFID vom Erkennungselement 23 der Beleuchtungsvorrichtung 1 erkannt wird - es erfolgt also eine RFID-Instrumentenerkennung. Die Regelungsschaltung ist dazu ausgebildet, den vom Erkennungselement 23 erkannten Instrumententyp zu erhalten und das zweite Regelsignal S2 basierend auf einem Vergleich des erkannten Instrumententyps mit Referenz-Instrumententypen, die in einer Look-Up Tabelle gespeichert sind, zu ermitteln. Im vorliegenden Fall ist das zweite Regelsignal S2 ein maximales Referenzsignal in Form einer maximalen Leistung $I_{PD-max}$ je Gauge-Grösse, welche als Stellgrösse zur Leistungsregelung an die Anregungsquelle 4, 4a und gegebenenfalls an die ergänzende Leuchtquelle 17, 17a übermittelt wird. Denkbar ist, dass dabei eine Führungsgrösse ermittelt und zur Regelung übermittelt wird, welche auf der maximalen Leistung $I_{PD-max}$ der verwendeten bzw. erkannten Gauge-Grösse sowie auf einem Einstellwert b, der von einem Benutzer einstellbar ist, erfolgen. Diese Führungsgrösse dient als Referenz-Strahlungsleistung $y_{SOLL}$, welche für die Regelung der Strahlungsleistung mittels der vom Sensorelement 21 erfassten Strahlungsleistung dient. Nebst der Strahlungsleistung berücksichtigt die Regelung hier auch Störgrössen, z.B. eine Temperatur des Sensorelements oder eine spektrale Empfindlichkeit des Sensorelements, welche zurückgeführt und bei der Ermittlung der Referenz-Strahlungsleistung $y_{SOLL}$ mitberücksichtigt werden.

Zusammengefasst lässt sich also festhalten, dass die erfindungsgemässe Beleuchtungsvorrichtung 1 viele Vorteile bietet. So gestattet sie eine Erhöhung des Lichtstroms, sodass bei 27G Operationen auch in widrigen Umständen (bspw. Netzhautblutungen) genügend Licht zur Operation im Auge zur Verfügung steht du sie gestattet eine Erhöhung des Lichtstroms, sodass Fasern 29 mit ≤200 μm zur Beleuchtung bei Vitrektomien verwendet werden können, ohne die Operationsbedingungen für den Chirurgen durch unzureichende Helligkeit zu erschweren (≥10 lm). Insbesondere erzeugt sie signifikant höheren Lichtstrom als bisher in Verkehr gebrachte Leuchtquellen bei 27G Vitrektomien (beispielsweise ≥25 lm statt bisher 8-11 lm) und liefert ausreichenden Lichtstrom, um sogar 29G Vitrektomien mit optimaler Helligkeit (≥10 lm) zu ermöglichen. Sie bietet eine erhöhte Patientensicherheit durch Erkennung des Lichtinstruments 2 und entsprechender Leistungsregelung des in die Faser 29 einzukoppelnden Lichtstroms. Weiter gestattet sie eine Speicherung und Anzeige der phototoxischen Dosis. Insbesondere gestattet sie durch Erkennung des Lichtinstrumentes 2 und Regelung des Lichtstroms identische Lichstromwerte (1-25 lm) für alle heute gängigen Gauge-Grössen (20G, 23G, 25G, 27G). Der Benutzer hat daher unabhängig von der Gauge-Grösse des verwendeten Lichtinstruments 2 immer optimalen/identischen Lichtstrom zur Verfügung. Sie gestattet die Integration von Photolumineszenz 7 in ein Lichtinstrument 2 für die Vitrektomie bei gleichzeitiger Farbhomogenität des Lichtkegels am distalen Faserende des Lichtinstruments 2. Sie gestattet eine Verringerung von Farbringen aufgrund chromatischer Aberration auch bei Verwendung unterschiedlich farbiger Leuchtquellen 4, 4a, 17, 17a. Zudem erlaubt sie eine Reduktion der phototoxischen Dosis auch bei Anregungsquellen 4, 4a oder ergänzenden Leuchtquellen 17, 17a in Form von blauen LED mit erhöhter Wellenlänge (λPeak ≥465 nm) im Vergleich zu der Wellenlänge der Laserdioden (λPeak = 450 nm) und sie ermöglicht eine lineare Farbverstellung.

BEZUGSZEICHENLISTE

1 ophthalmische Beleuchtungsvorrichtung
2 ophthalmisches Lichtinstrument
3 Leuchtelement
4 Anregungsquelle
5 Anregungslicht
6 Beleuchtungslicht
7 Photolumineszenz
8 Fläche von Leuchtelement
9 Frontseite von Leuchtelement
10 Rückseite von Leuchtelement
11 Wärmeleitelement
12 Kühlelement
13 Gehäuse
14 optisches Filterelement
15 Reflexionslicht
16 Transmissionslicht
17 ergänzende Leuchtquelle
18 Ergänzungslicht
19 Kollektorlinse
20 Kondensorlinse
21 Sensorelement
22 Randbereich
23 Erkennungselement
24 erkennbares Element
25 Leuchtquelle
26 Stecker
27 Buchse
28 Handgriff
29 Lichtleiter
30 Kanüle
31 Anschlagsfläche
32 Anschlagsfläche
33 Lüfter
34 Frontfläche
35 Rückfläche
36 Kollimationslinse
37 Fokuslinse
38 Kollimationslinse
39 achromatisches Linsenpaar

$\alpha$ Winkel

A Ausbreitungsrichtung
A1 Ausbreitungsrichtung
A2 Ausbreitungsrichtung
D Dickenrichtung
O optische Achse
S Regelsignal
S2 Regelsignal
M Mittelachse
b Einstellwert
$y_{IST}$ erfasste Strahlungsleistung
$y_{SOLL}$ Referenz-Strahlungsleistung
$I_{Laser}$ Stellgrösse
$I_{LED}$ Stellgrösse
$I_{PD-max}$ Stellgrösse

100 ophthalmisches Beleuchtungssystem

**Patentansprüche**

1. Ophthalmische Beleuchtungsvorrichtung (1) zur Beleuchtung eines ophthalmischen Lichtinstruments (2) umfassend:

   - mindestens ein Leuchtelement (3), und
   - mindestens eine Anregungsquelle (4),

   **dadurch gekennzeichnet,**

   **dass** die Anregungsquelle (4) zur Anregung des Leuchtelements (3) mittels Anregungslicht (5) aus stimulierter Emission ausgebildet ist, und **dass** das Leuchtelement (3) dazu ausgebildet ist, nach Anregung zu photolumineszieren, und wobei die ophthalmische Beleuchtungsvorrichtung (1) dazu ausgebildet ist, das ophthalmische Lichtinstrument (2) mittels Beleuchtungslicht (6) umfassend oder bestehend aus Photolumineszenz (7) und/oder stimulierter Emission (5) zu beleuchten.

2. Ophthalmische Beleuchtungsvorrichtung (1) gemäss Anspruch 1, wobei die Anregungsquelle (4) und/oder das Anregungslicht (5) in einem Winkel ($\alpha$) zum Leuchtelement (3), insbesondere zu einer Fläche (8) des Leuchtelements (3), angeordnet sind, und wobei der Winkel ($\alpha$) vorzugsweise etwa 30° oder mehr, beispielsweise 45° oder mehr wie etwa 90° beträgt.

3. Ophthalmische Beleuchtungsvorrichtung (1) gemäss einem der vorhergehenden Ansprüche, wobei das Leuchtelement (3) eine Frontseite (9) und eine entgegengesetzte Rückseite (10) aufweist,

   wobei das Leuchtelement (3) derart angeordnet ist, dass sich die Photolumineszenz (7) ausgehend von der Frontseite (9) des Leuchtelements (3) entlang einer Ausbreitungsrichtung (A) ausbreitet, und wobei die Anregungsquelle (4) bezüglich der Ausbreitungsrichtung (A) gesehen vor dem Leuchtelement (3) oder nach dem Leuchtelement (3) angeordnet ist.

4. Ophthalmische Beleuchtungsvorrichtung (1) gemäss einem der vorhergehenden Ansprüche, wobei das Leuchtelement (3):

   - schichtförmig ausgebildet ist, und/oder

- mindestens ein photolumineszierendes Material umfasst oder daraus besteht.

5. Ophthalmische Beleuchtungsvorrichtung (1) gemäss einem der vorhergehenden Ansprüche, wobei die Anregungsquelle (4) und das Leuchtelement (3) derart angeordnet und ausgebildet sind, dass ein Kombinationsspektrum umfassend i) das Photolumineszenz-Spektrum des Leuchtelements (3) und ii) durch das Leuchtelement (3) transmittiertes Anregungslicht (5) und/oder vom Leuchtelement (3) reflektiertes Anregungslicht (5) gebildet wird, wobei das Kombinationsspektrum aus dem transmittierten oder reflektierten Anregungslicht (5) und der Photolumineszenz (7) besteht und vorzugsweise Weisslicht bildet.

6. Ophthalmische Beleuchtungsvorrichtung (1) gemäss einem der vorhergehenden Ansprüche, umfassend mindestens ein Wärmeleitelement (11) und mindestens ein Kühlelement (12), wobei das Wärmeleitelement (11) zum Leiten einer vom Anregungslicht erzeugten Wärme zum Kühlelement (12) ausgebildet ist, und wobei das Kühlelement (12) zum Abgeben der Wärme an die Umgebung ausgebildet ist.

7. Ophthalmische Beleuchtungsvorrichtung (1) gemäss einem der vorhergehenden Ansprüche, umfassend mindestens ein optisches Filterelement (14),

   wobei das optische Filterelement (14) dazu ausgebildet ist, einfallendes Licht umfassend die Photolumineszenz (7) und optional zumindest teilweise das Anregungslicht (5) basierend auf mindestens einer optischen Eigenschaft des einfallenden Lichts, insbesondere der Wellenlänge, als Reflexionslicht (15) zu reflektieren und als Transmissionslicht (16) zu transmittieren, und
   wobei das Beleuchtungslicht (6) das Reflexionslicht (15) oder das Transmissionslicht (16) umfasst oder daraus besteht, und/oder
   wobei das optische Filterelement (14) dazu ausgebildet ist, das Anregungslicht (5) zumindest teilweise aus dem Beleuchtungslicht (6) zu filtern.

8. Ophthalmische Beleuchtungsvorrichtung (1) gemäss Anspruch 7, umfassend mindestens eine ergänzende Leuchtquelle (17) zur Aussendung von Ergänzungslicht (18) auf das optische Filterelement (14) derart, dass ein ergänztes Kombinationsspektrum umfassend i) das Reflexionslicht (15) oder das Transmissionslicht (16) und ii) das Ergänzungslicht (18) gebildet wird, und

   wobei das Beleuchtungslicht (6) das ergänzte Kombinationsspektrum umfasst oder daraus besteht, und
   wobei eine Wellenlänge des Ergänzungslichts (18) vorzugsweise veränderbar ist und/oder gleich wie oder verschieden von einer Wellenlänge des Anregungslichts (5) ist.

9. Ophthalmische Beleuchtungsvorrichtung (1) gemäss einem der vorhergehenden Ansprüche, wobei das aus der ophthalmischen Beleuchtungsvorrichtung (1) abgestrahlte Beleuchtungslicht (6) einen objektseitigen Abstrahlwinkel ($\varphi$) bildet,

   wobei das durch die ophthalmische Beleuchtungsvorrichtung (1) zu beleuchtende ophthalmische Lichtinstrument (2) einen bildseitigen Abstrahlwinkel ($\varphi'$) definiert, und
   wobei die ophthalmische Beleuchtungsvorrichtung (1) derart ausgebildet ist, dass ein Abbildungsmassstab (M) der ophthalmischen Beleuchtungsvorrichtung (1) einen Referenz-Abbildungsmassstab nicht überschreitet und/oder unterschreitet, und/oder
   wobei die ophthalmische Beleuchtungsvorrichtung (1) derart ausgebildet ist, dass der bildseitige Abstrahlwinkel ($\varphi'$) des ophthalmischen Lichtinstruments (2) nicht überschritten und/oder unterschritten wird.

10. Ophthalmische Beleuchtungsvorrichtung (1) gemäss einem der vorhergehenden Ansprüche, umfassend mindestens eine Kollektorlinse (19) und eine Kondensorlinse (20),

    wobei die Kollektorlinse (19) derart angeordnet und ausgebildet ist, dass sie die divergente Photolumineszenz (7) des Leuchtelements (3) zumindest teilweise entlang einer optischen Achse (O) ausrichtet,
    wobei die Kondensorlinse (20) derart angeordnet und ausgebildet ist, dass sie das Beleuchtungslicht (6) aus der Beleuchtungsvorrichtung (1) und insbesondere in ein ophthalmisches Lichtinstrument (2) ausgibt, und
    wobei die Kollektorlinse (19) und die Kondensorlinse (20) jeweils ein achromatisches Linsenpaar sind.

11. Ophthalmische Beleuchtungsvorrichtung (1) gemäss einem der vorhergehenden Ansprüche, umfassend mindestens ein Sensorelement (21) zur Erfassung einer Strahlungsleistung des Beleuchtungslichts (6) und/oder des Anregungslichts (5) und/oder des Ergänzungslichts (18), und

    wobei das Sensorelement (21) teilweise in einem Strahlengang des Beleuchtungslichts (6) und/oder des Anregungslichts (5) und/oder des Ergänzungslichts (18) angeordnet ist, und/oder wobei die ophthalmische Beleuchtungsvorrichtung (1) dazu ausgebildet ist, ein auf der erfassten Strahlungsleistung basiertes Regelsignal (S) an die Anregungsquelle (4) und/oder die ergänzende Leuchtquelle (17) zur Leistungsregelung der Anregungsquelle (4) und/oder der ergänzenden Leuchtquelle (17) zu senden.

12. Ophthalmische Beleuchtungsvorrichtung (1) gemäss einem der vorhergehenden Ansprüche, umfassend mindestens ein Erkennungselement (23) zum Erkennen eines erkennbaren Elements (24) eines mit der ophthalmischen Beleuchtungsvorrichtung (1) verbundenen ophthalmischen Lichtinstruments (2), und

    wobei die ophthalmische Beleuchtungsvorrichtung (1) dazu ausgebildet ist, ein auf dem erkannten erkennbaren Element (24) basiertes Regelsignal (S2) an die Anregungsquelle (4) und/oder die ergänzende Leuchtquelle (17) zur Leistungsregelung der Anregungsquelle (4) und/oder der ergänzenden Leuchtquelle (17) zu senden, und
    wobei das erkennbare Element (24) vorzugsweise auf einem Instrumententyp des ophthalmischen Lichtinstruments (2) basiert.

13. Ophthalmische Beleuchtungsvorrichtung (1) zur Beleuchtung eines ophthalmischen Lichtinstruments (2) umfassend:

    - mindestens eine Leuchtquelle (25) zur Erzeugung von Beleuchtungslicht (6), und
    - mindestens ein Sensorelement (21) zur Erfassung einer Strahlungsleistung des Beleuchtungslichts (6),

**dadurch gekennzeichnet,**

    **dass** das Sensorelement (21) teilweise in einem Strahlengang des Beleuchtungslichts (6) angeordnet ist, und
    **dass** die ophthalmische Beleuchtungsvorrichtung (1) dazu ausgebildet ist, ein auf der erfassten Strahlungsleistung basiertes Regelsignal (S) an die Leuchtquelle (25) zur Leistungsregelung der Leuchtquelle (25) zu senden.

14. Ophthalmische Beleuchtungsvorrichtung (1) zur Beleuchtung eines ophthalmischen Lichtinstruments (2) umfassend:

    mindestens eine Leuchtquelle (25) zur Erzeugung von Beleuchtungslicht (6), **dadurch gekennzeichnet,**

    **dass** die ophthalmische Beleuchtungsvorrichtung (1) weiter mindestens ein Erkennungselement zum Erkennen eines erkennbaren Elements (24) eines mit der ophthalmischen Beleuchtungsvorrichtung (1) verbundenen ophthalmischen Lichtinstruments (2) aufweist, und
    **dass** die ophthalmische Beleuchtungsvorrichtung (1) dazu ausgebildet ist, ein auf dem erkannten erkennbaren Element (24) basiertes Regelsignal (S2) an die Leuchtquelle (25) zur Leistungsregelung der Leuchtquelle (25) zu senden, und
    wobei das erkennbare Element (24) vorzugsweise auf einem Instrumententyp des ophthalmischen Lichtinstruments (2) basiert.

15. Ophthalmisches Beleuchtungssystem (100) umfassend eine ophthalmische Beleuchtungsvorrichtung (1) gemäss einem der vorhergehenden Ansprüche und ein ophthalmisches Lichtinstrument (2), wobei das ophthalmische Lichtinstrument (2) zum Zuleiten von Beleuchtungslicht (6) in den intraokularen Raum ausgebildet ist.

100

1

33

23

32

26

2

31

27

13

30    28    29

A; O

**FIG. 1**

**FIG. 2**

**FIG. 3**

**FIG. 4**

1

13

**FIG. 5**

17

38

1

18

39

37

19    13    36

A; O

20

A1    14

**FIG. 6**

FIG. 7

FIG. 8

**FIG. 9**

rel. Lichtstrom [lumen/nm]

Wellenlänge [nm]

**FIG. 10**

100

2

1

A; M

6

φ'

20

21

19

5

7

4; 25

4a; 25a

5a

φ

φt

φt

5

10; 35    3

8; 9; 34

11

12

D

**FIG. 11**

FIG. 12

FIG. 13

FIG. 14

Abbildungsmassstab

——— 500um
··········· 400um
– – – 300um
–·– 200um
—·· 100um
——— 700um
– – – – 20lm @ 5100 K (4% Blauanteil)

**FIG. 15**

FIG. 16

**FIG. 17**

FIG. 18

**FIG. 19**

**FIG. 20**

Europäisches
Patentamt
European
Patent Office
Office européen
des brevets

## EUROPÄISCHER RECHERCHENBERICHT

Nummer der Anmeldung

EP 24 17 4607

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 10 117 578 B2 (IP LIBERTY VISION CORP [US]) 6. November 2018 (2018-11-06) | 1-4,6-15 | INV. A61B3/00 |
| Y | * Zusammenfassung * * Ansprüche 1-12; Abbildungen 1-4 * * Spalte 2, Zeile 49 – Zeile 52 * * Spalte 3, Zeile 36 – Zeile 43 * * Spalte 4, Zeile 9 – Zeile 45 * ----- | 5 | A61B5/00 |
| Y | US 2022/307983 A1 (LAPOINTE NICOLAS [CA] ET AL) 29. September 2022 (2022-09-29) | 5 | |
| A | * Zusammenfassung * * Absatz [0005] – Absatz [0102] * ----- | 1,15 | |
| A | AU 2011 289 406 A1 (ALCON RES LTD) 28. Februar 2013 (2013-02-28) * das ganze Dokument * ----- | 1-15 | |

RECHERCHIERTE SACHGEBIETE (IPC)

A61B

Der vorliegende Recherchenbericht wurde für alle Patentansprüche erstellt

| Recherchenort | Abschlußdatum der Recherche | Prüfer |
|---|---|---|
| München | 10. Oktober 2024 | Tommaseo, Giovanni |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**
EP 24 17 4607

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-10-2024

| Im Recherchenbericht angeführtes Patentdokument | Datum der Veröffentlichung | Mitglied(er) der Patentfamilie | Datum der Veröffentlichung |
|---|---|---|---|
| US 10117578 B2 | 06-11-2018 | AU 2014374326 A1 | 04-08-2016 |
| | | CA 2935666 A1 | 09-07-2015 |
| | | CN 106029020 A | 12-10-2016 |
| | | EP 3089717 A1 | 09-11-2016 |
| | | ES 2717258 T3 | 20-06-2019 |
| | | JP 6532470 B2 | 19-06-2019 |
| | | JP 2017502765 A | 26-01-2017 |
| | | PL 3089717 T3 | 30-08-2019 |
| | | US 2015182152 A1 | 02-07-2015 |
| | | US 2019239743 A1 | 08-08-2019 |
| | | WO 2015102800 A1 | 09-07-2015 |
| US 2022307983 A1 | 29-09-2022 | CA 3140557 A1 | 10-12-2020 |
| | | CN 114173664 A | 11-03-2022 |
| | | EP 3979914 A1 | 13-04-2022 |
| | | JP 7510441 B2 | 03-07-2024 |
| | | JP 2022535434 A | 08-08-2022 |
| | | US 2022307983 A1 | 29-09-2022 |
| | | WO 2020243842 A1 | 10-12-2020 |
| AU 2011289406 A1 | 28-02-2013 | AU 2011289406 A1 | 28-02-2013 |
| | | BR 112013003344 A2 | 12-07-2016 |
| | | CA 2808071 A1 | 16-02-2012 |
| | | CN 103124532 A | 29-05-2013 |
| | | EP 2603151 A1 | 19-06-2013 |
| | | ES 2527667 T3 | 28-01-2015 |
| | | JP 5848348 B2 | 27-01-2016 |
| | | JP 2013537462 A | 03-10-2013 |
| | | US 2012041461 A1 | 16-02-2012 |
| | | WO 2012021628 A1 | 16-02-2012 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20130265548 A1